# EUROPÄISCHE PATENTSCHRIFT

(11) **EP 2 857 493 B1**
(45) Veröffentlichungstag und Bekanntmachung des Hinweises auf die Patenterteilung: **01.05.2019**
(21) Anmeldenummer: 14186392.8
(22) Anmeldetag: 25.09.2014
(51) Int. Cl.: C12M 1/107, C12M 1/00, C12M 3/00, C12M 1/06

(54) **Rührwerk für einen Behälter, Behälter sowie Verfahren für den Einbau eines Rührwerks in einen Behälter**
Agitator for a container, container and method for integrating an agitator into a container
Agitateur pour un récipient, récipient et procédé de montage d'un agitateur dans un récipient

(30) Priorität: 01.10.2013 DE 102013219938
(43) Veröffentlichungstag der Anmeldung: 08.04.2015
(73) Patentinhaber: Peters, Jean-Marc, 4700 Eupen (BE)
(72) Erfinder: Peters, Jean-Marc, 4700 Eupen (BE)
(74) Vertreter: Bauer, Dirk

(56) Entgegenhaltungen:
- EP-A1- 2 636 444
- WO-A2-2007/110775
- DE-A1-102004 027 077
- DE-A1-102009 041 569
- DE-A1-102010 053 242
- DE-U1- 8 312 809
- DE-U1-202004 004 101
- DE-U1-202006 011 144
- DE-U1-202013 006 429

## Beschreibung

Die vorliegende Erfindung betrifft ein Set gemäß dem Oberbegriff von Anspruch 1, umfassend einen Fermenter und ein Rührwerk.

Ferner betrifft die Erfindung einen Behälter gemäß dem Oberbegriff von Anspruch 4.

Abschließend betrifft die Erfindung ein Verfahren gemäß dem Oberbegriff von Anspruch 16.

### Stand der Technik

Rührwerke und Behälter, insbesondere Fermenter der eingangs beschriebenen Art sind gemäß dem Stand der Technik bereits seit geraumer Zeit bekannt und kommen vor allem im Bereich der Biogasanlagen zum Einsatz, die zur Erzeugung regenerativer Energie genutzt werden.

Rührwerke der eingangs beschriebenen Art unterliegen im Betrieb einem gewissen normalen Verschleiß. Dieser betrifft in erster Linie die Lager und das Getriebe der Rührwerke. In Abhängigkeit von der Qualität des Rührwerks kommt es früher oder später zu dessen Ausfall aufgrund eines Versagens mindestens eines der Verschleißteile, es sei denn, es findet bereits zuvor ein vorsorglicher Austausch von Verschleißteilen statt. Im Falle eines Versagens müssen die Verschleißteile sodann gewechselt werden, um das Rührwerk wieder in Betrieb nehmen zu können. Bei Fermentern mit mehreren Rührwerken ist ein temporärer Ausfall eines einzelnen Rührwerks mitunter für eine gewisse Zeit tolerabel. Durch die sinkende Umwälzung des in dem Fermenter befindlichen Substrats erlahmt jedoch der gewollte biologische Zersetzungsprozess, was letztendlich zu einer Reduktion des Umsatzes sowie des Energieoutputs der Biogasanlage führt. Durch derartige Einbußen wird die Wirtschaftlichkeit der Biogasanlage empfindlich reduziert. Ein längerfristiger Ausfall eines Rührwerks oder gar mehrerer Rührwerke ist folglich nicht akzeptabel.

Daher werden die ausgefallenen Rührwerke repariert oder ausgetauscht. Hierzu ist es regelmäßig notwendig, den Fermenter zu öffnen, zu entleeren und den Betrieb der Biogasanlage einzustellen. Hierdurch verursachte Stillstandszeiten erzeugen beträchtliche Kosten, die gerne vermieden werden würden.

Eine Lösung dieses Problems ergibt sich beispielsweise aus der EP 2 064 308 A1, die ein Rührwerk beschreibt, das im laufenden Betrieb des Fermenters gewechselt werden kann. Dies wird dadurch erreicht, dass ein unteres, einer Bodenplatte des Fermenters zugewandtes Ende der Welle des Rührwerks welches Ende zusammen mit einem Drehlager in Form einer in die hohle Welle eingesetzten Lagerpatrone die Lagereinrichtung bildet, "lose" in eine Aufnahmeeinrichtung des Fermenters eingesetzt ist. Diese Aufnahmeeinrichtung ist dazu geeignet, ein Verdrehen der Lagereinrichtung des Rührwerks, die in Form eines Wälzlagers ausgeführt ist, zu blockieren und es auf diese Weise zu lagern. Allerdings kann das Rührwerk ohne Weiteres aus der Aufnahmeeinrichtung herausgehoben werden, ohne dass hierfür besondere Maßnahmen ergriffen werden müssen. Insbesondere ist es nicht notwendig, das Rührwerk an dessen unterem Ende von der Aufnahmeeinrichtung mittels Werkzeugen oder dergleichen zu lösen.

Die bekannte Konstruktion ist jedoch insoweit nachteilig, als sie zum einen zwingend eine vertikale Betriebsposition der Rührwerkswelle erfordert und zum anderen zwingend einen solchen Fermenter benötigt, dessen Deckeneinrichtung von einer starren Betondecke gebildet ist. Diese dient als Rahmenkonstruktion für die Aufnahme der Rührwerke, die von der Oberseite her in den Fermenter herabgelassen werden. Eine solche Deckenkonstruktion ist insoweit nachteilig, als sie zum einen einen hohen Planungsaufwand erfordert und für jeden Fermenter eines jeden Projekts gesondert kalkuliert und geplant werden muss und zum anderen die Errichtung des Fermenters verzögert, da eine solche Deckenkonstruktion in der Herstellung vergleichsweise aufwendig ist.

Unter diesem Gesichtspunkt sind solche Fermenter mit Membranabschlüssen deutlich von Vorteil. Solche Membrane werden gasdicht an die umlaufenden Wandungen des Fermenters angeschlossen und wölben sich sodann aufgrund des sich in dem Innenraum des Fermenters bildenden Biogases nach außen und bilden gewissermaßen eine Art Zeltdach über dem Fermenter. Die Errichtung einer solchen Deckeneinrichtung in Form einer flexiblem Membran ist folglich erheblich schneller und kostengünstiger möglich. Außerdem entfällt der beschriebene Planungsaufwand in wesentlichen Teilen. Allerdings ist ein Einsatz vertikal orientierter Rührwerke in solchen Anlagen nicht möglich, da eine Lagerung des Rührwerks an dessen oberen Ende ohne eine Rahmenkonstruktion nicht ohne Weiteres erfolgen kann. Die Membran selbst ist nämlich nicht geeignet, auftretende Lagerkräfte aufzunehmen.

Daher werden Fermenter, die mit einer solchen Membran verschlossen sind, typischerweise mit Rührwerken oder sonstigen Vorrichtungen, die zum Umwälzen des Substrats geeignet sind, betrieben, die seitlich durch die Wandung in den Fermenter eingesetzt werden. Hierfür können sowohl schräge Rührwerke als auch sogenannte "Stabmixer" zum Einsatz kommen. Schräge Rührwerke sind beispielsweise den Schriften DE 20 2004 004 101 U1 und WO 2007/110775 A2 entnehmbar. Diese zeigen Rührwerke, die seitlich durch die Wandungen des jeweiligen Fermenters aus dem Innenraum nach außen geführt sind und in bzw. an den Wandungen gelagert sind. Die gezeigten Systeme weisen allerdings den Nachteil auf, dass der Fermenter nur bis zu einer relativ kleinen Füllhöhe befüllt werden kann, die in jedem Fall unterhalb der seitlichen Montageöffnung in der Wandung des Fermenters endet. Die Höhe des Fermenters kann mithin nur zum Teil ausgenutzt werden. Ferner ergibt sich das Problem, dass aufgrund der konstruktionsbedingten Nähe der Welle des Rührwerks zur Wandung des Fermenters im oberen Endabschnitt des Rührwerks die zugehörigen Rührelemente sehr nah an den Wandungen angeordnet sind. Diese müssen jedoch zumindest so weit von der Wandung entfern angeordnet sein, dass sie bei einer Rotation der Welle des Rührwerks nicht gegen die Wandung schlagen.

Darüber hinaus ist aus der DE 10 2004 027 077 A1 ein beheizbares Rührwerk für Fermentationsbehälter bekannt, das zum Eintrag sowohl mechanischer als auch thermischer Energie in einen Fermentationsbehälter vorgesehen ist. Das bekannte Rührwerk besitzt eine beheizbare Rührwelle und an der Rührwelle befestigte Wärmeleitflächen, an denen wiederum Rührblätter angeordnet sind. In der Einbauposition ist die Wellenachse gegenüber der Vertikalen um einen Winkel von ca. 45° geneigt. Die Wellenlagerung ist in einem behälterfesten Lagerfuß untergebracht (so genannter Montagebock). Der Neigungswinkel der unteren behälterfesten Lagereinrichtung ist im Zuge der Montage bei leerem Fermentationsbehälter einstellbar. Nach Verschleiß des Lagers ist der Behälter zu entleeren, um die Lagereinrichtung austauschen zu können.

Dasselbe Problem einer zwingend erforderlichen Behälter- bzw. Reaktorentleerung besteht bei der Rührvorrichtung gemäß der DE 10 2007 034 463 A1. Ein Unterteil der Lagereinrichtung ist fest mit dem dortigen Behälterboden verbunden, so dass ein Austausch bei gefülltem Reaktor nicht möglich ist. Darüber hinaus ist das vorbenannte Rührwerk in seiner Einbauposition vertikal ausgerichtet.

Die DE 82 12 809 U1 offenbart eine Rührvorrichtung, bei der eine bodenfeste Lagereinrichtung vorhanden ist. Die Aufgabe der in diesem Dokument beschriebenen Erfindung besteht darin, eine Strömungsrichtung in dem zu rührenden Substrat zu erreichen, die parallel zu der Rührwerkswelle ausgerichtet ist. Treten Schäden an der bodenseitigen Lagereinrichtung auf, so ist der Behälter zu entleeren, um einen Austausch der Lagereinrichtung vornehmen zu können.

Auch aus der DE 20 2006 011 144 U1 ist ein in seiner Einbauposition schräg gestelltes Rührwerk mit einer behälterfest angeordneten Lagereinrichtung entnehmbar. Hieraus ergeben sich die bereits zuvor genannten Probleme bei einem Lagerschaden bzw. einem turnusmäßig vorgesehenen Lageraustausch.

Aus der DE 20 2009 010 167 U1 ist ein Rührwerk der so genannten Tauchmotor-Bauweise bekannt, bei dem ein Rührwerksmast drehbar auf einem Zapfen am Boden gelagert ist und mithilfe einer ersten Kurbel in Rotation versetzbar ist. Mithilfe eines Zugseils kann das Rührwerk, das an einer Hülse in Längsrichtung des Mastes verschiebbar an diesem gelagert ist, bedarfsweise aus der Fermentationsmasse entnommen werden bzw. in diese abgesenkt werden. Eine Schrägstellung des Rührwerks in seiner Betriebsposition ist weder vorgesehen noch sinnvoll. Auch die EP 2 636 444 A1 offenbart ein derartiges Tauchmotorrührwerk.

Schließlich offenbart die WO 2011/139209 A1 noch ein hängendes Rührwerk, dessen unteres Ende mittels einer federbelasteten Kette an dem Behälterboden abgespannt ist. Zum einen ist bei einem Lagerverschleiß an der Unterseite der Rührwerkswelle eine Fermenterentleerung erforderlich und zum anderen erfordert die hängende Lagerung zur Aufnahme der auf das Rührwerk wirkenden Zugkräfte eine sehr belastbare Konstruktion der oberen Rührwerksbefestigung, die typischerweise nur in Form einer Betondecke des Fermentationstanks ausgeführt werden kann. Eine Schrägstellung des Rührwerks ist nicht vorgesehen.

### Aufgabe

Aufgabe der vorliegenden Erfindung ist es demzufolge, ein Rührwerk und einen Behälter, insbesondere Fermenter bereitzustellen, die es erlauben, das Rührwerk möglichst einfach im Betrieb zu wechseln und dabei eine Lagerung zu verwenden, die einen möglichst einfachen Aufbau der Deckeneinrichtung des Behälters erlaubt.

### Lösung

Die zugrunde liegende Aufgabe wird erfindungsgemäß durch ein Set mit den Merkmalen des Anspruchs 1 gelöst.

Die Erfindung wird nachfolgend beispielhaft am Beispiel eines mit einem Rührwerk ausgestatteten Fermenters, insbesondere für Biogasanlagen, beschrieben, ohne hierdurch eine Einschränkung auf diesen Einsatzfall zu bezwecken. Vielmehr ist das Rührwerk auch für eine Vielzahl anderer Rührwerkaufgaben geeignet.

Wie bereits angedeutet, ist die Erfindung aus dem Wunsch heraus hervorgebracht worden, eine Lagerung der Rührwerke eines Behälters, insbesondere eines Fermenters, zu vereinfachen. Diese Lagerung umfasst in jedem Fall ein oberes Ende des Rührwerks, der befestigt werden muss, um Betriebskräfte des Rührwerks abzutragen. Dieser Lastabtrag ist umso aufwendiger zu bewerkstelligen, je weiter das zu lagernde obere Ende eines jeweiligen Rührwerks von der Wandung des zugehörigen Fermenters entfernt angeordnet ist. Dies ergibt sich daraus, dass die abzutragenden Kräfte über umso weitere Strecken bis in die Wandung des jeweiligen Fermenters abgetragen werden müssen. Folglich ergibt sich der Wunsch, das obere Ende des Rührwerks möglichst nah an der Wandung anzuordnen, möglicherweise sogar "in" der Wandung, das heißt die Lagerkräfte direkt in die Wandung einzuleiten. Daher ist es grundsätzlich denkbar, das obere Ende des Rührwerks an einer "Oberseite" oder "seitlich" aus dem jeweils zugehörigen Fermenter herauszuführen. Beide Varianten können je nach Art und Größe des Fermenters vorteilhaft sein. Eine Lagerung des Rührwerks möglichst nahe zur Wandung des Fermenters birgt jedoch zwei Nachteile: Zum einen besteht - wie in der Beschreibung zum Stand der Technik bereits angedeutet - das Risiko, dass Rührelemente des Rührwerks gegen die Wandung des Fermenters schlagen. Folglich muss ein gewisser Mindestabstand zwischen beiden Teilen immer gewährleistet sein. Zum anderen ist bei einer wandungsnahen Anordnung des Rührwerks dessen Rührwirkung auf einen Randbereich des Fermenters beschränkt, während in einem Mittelbereich des Fermenters befindliches Substrat im Wesentlichen ungerührt bleibt.

Für das erfindungsgemäße Set ergibt sich aus der vorgenannten Überlegung der Wunsch, dieses in seiner Einbau- bzw. Betriebsposition, in der es vollständig in einem jeweiligen Fermenter montiert ist, möglichst in einem gewissen Mindestabstand zu der Wandung des Fermenters zu positionieren, damit das mindestens eine Rührelement des Rührwerks genügend Abstand zur jeweilig nahesten Wandung des Fermenters hat und nicht Gefahr läuft, gegen selbige zu schlagen. Außerdem ist es grundsätzlich erwünscht, die Rührwirkung des Rührwerks nicht nur auf Randbereiche des Fermenters zu beschränken, sondern diese auch in einem Mittelbereich des Fermenters zu erzielen. Gleichzeitig ist es ebenso erstrebenswert, das obere Ende des Rührwerks nicht zu weit von der Wandung des Fermenters entfernt anzuordnen, da in einem solchen Fall eine "starrer Deckeneinrichtung", die zum Abtrag der Lagerkräfte des oberen Endes des Rührwerks bis in die Wandung des Fermenters geeignet ist, ebenso weit von der Wandung des Fermenters weggeführt werden müsste, um das obere Ende des Rührwerks aufzunehmen. Eine flexible Deckeeinrichtung, beispielsweise in Form einer flexiblen Membran, ist für die Aufnahme von Lagerkräften eines Rührwerks nicht geeignet. Doch allein die Aufnahme der Lagerkräfte des Rührwerks mittels der starren Deckeneinrichtung genügt nicht. Diese Lagerkräfte müssen sodann von der Deckeneinrichtung in jedem Fall bis in die Wandung des Fermenters abgeleitet werden. Dies fällt umso schwerer, desto weiter das obere Ende des Rührwerks von der Wandung entfernt angeordnet ist und umso weiter die Lagerkräfte von der starren Deckeneinrichtung geleitet werden müssen. Somit ergibt sich das Dilemma, zwar zum einen wegen der Rührwirkung im Mittelbereich des Fermenters das Rührwerk gern möglichst weit entfernt von der Wandung des Fermenters anzuordnen, es gleichzeitig aber wegen seiner Lagerung nicht zu weit von der Wandung zu entfernen.

Der erfindungsgemäßen Lösung liegt schließlich die Idee zugrunde, das Rührwerk im Zuge seiner Montage zunächst möglichst nah an der Wandung (und möglichst an einem Hebewerkzeug hängend sowie möglichst in vertikale Richtung) in den Behälter, insbesondere den Fermenter einzusetzen. Die Montageposition ist erreicht, sobald das untere Ende der Rührwerkswelle mit der dort befindlichen Gleit- oder Wälzlagerung im Zuge des Absenkens die Führungseinrichtung berührt und sodann lediglich das untere Ende des Rührwerks möglichst weit von der Wandung weg zu bewegen, während das obere Ende des Rührwerks zumindest in Wandnähe verbleibt. Die erfindungsgemäße Lösung beinhaltet demzufolge ein in dessen endgültiger Betriebsposition schräg orientiertes Rührwerk, dessen Längsachse der Welle gegen eine Vertikale geneigt angeordnet ist. Die für die Bewegung des unteren Endes der Rührwerkswelle benötigte Energie kann durch Umwandlung aus potentieller Energie "erzeugt" werden, wenn die Führungseinrichtung in Richtung auf die Aufnahmeeinrichtung zu abfallend ausgeführt ist. Bei dieser Lösung ist eine mittels des mindestens einen Rührelements erzielte Rührwirkung auch außerhalb des Randbereichs des Fermenters vorhanden, da sich das Rührwerk aufgrund seiner schrägen Stellung zumindest teilweise in Richtung des Mittelbereichs des Fermenters erstreckt und hier eine Rührung des Substrats bewirken kann. Gleichzeitig muss eine Deckeneinrichtung, die für die Aufnahme des oberen Endes des Rührwerks vorgesehen ist, lediglich in einem Randbereich des Fermenters starr ausgebildet sein , wobei sich eine solche starre Deckeneinheit nicht in erheblichem Maße ausgehend von der Wandung in Richtung des Mittelbereichs des Fermenters erstrecken muss. Ein restlicher Teil der Deckeneinrichtung kann währenddessen von der flexiblen Deckeneinheit, insbesondere einer flexiblen Membran gebildet werden. Möglicherweise ist sogar eine Lagerung des oberen Endes des Rührwerks direkt in der Wandung des Fermenters möglich, so dass die starre Deckeneinheit nahezu vollständig entfallen kann, wobei lediglich gegebenenfalls ein Abschnitt verbleiben muss, der zur Montage bzw. Demontage des Rührwerks verwendet wird. Dies ist insbesondere bei hohen Fermentern (typischerweise höher als 8 m) denkbar, die nicht über ihre gesamte Höhe mit Substrat gefüllt sind und bei denen eine seitliche Befestigung des oberen Endes des Rührwerks folglich keine Nachteile hätte. In diesem Fall kann die Deckeneinrichtung des Fermenters entsprechend sogar fast vollständig von einer flexiblen Membran gebildet werden.

Die von der Wandung des Fermenters weg gerichtete Bewegung des Rührwerks während des Montagevorgangs erfolgt erfindungsgemäß mittels der Gleitlagerung oder der Rollenlagerung der Lagereinrichtung. Die Gleiteinrichtung weist eine Gleitfläche auf, die besonders gut geeignet ist, auf einer korrespondierenden Gleitfläche gleitreibungsarm zu gleiten. Insbesondere sollte die Gleitfläche möglichst glatt sein. Alternativ ist die genannte Rollenlagerung möglich, die mindestens eine Laufrolle aufweist, die geeignet ist, auf einer korrespondierenden Abrollfläche abzurollen. Als solche ist beispielsweise eine Oberfläche der Bodenplatte des Fermenters vorstellbar. Gleichwohl ist es kaum vorstellbar, das Rührwerk mit seiner hohen Masse zielgerichtet über eine im Wesentlichen ebene Oberfläche der Bodenplatte des Fermenters zu bewegen, zumal ein Einbau des Rührwerks auch dann möglich sein muss, wenn der Fermenter gefüllt ist, sodass in dem Fermenter befindliches Substrat einen erheblichen Widerstand gegen eine angestrebte Bewegung des unteren Endes des Rührwerks bewirkt Vorteilhafterweise verfügt der Fermenter daher über eine Führungseinrichtung, mittels derer die Gleitfläche der Gleitlagerung bzw. die Laufrolle der Rollenlagerung - und mithin die Lagereinrichtung sowie letztendlich das gesamte Rührwerk - besonders einfach geführt werden kann. Eine solche Variante ist nachstehend ausführlich beschrieben.

Letztendlich wird angestrebt, die Lagereinrichtung des Rührwerks mittels der Gleit- bzw. der Rollenlagerung in eine Einbauposition zu bewegen, in der die Lagereinrichtung anschließend verbleibt und das Rührwerk in Betrieb genommen werden kann. Von der Wirkung her ist dieser Ansatz mit der EP 2 064 308 B1 vergleichbar. Somit ist es von besonderem Vorteil, wenn die Lagereinrichtung mittels der Gleit- bzw. Rollenlagerung in eine Aufnahmeeinrichtung "einfahrbar" oder "einschiebbar" ist, wobei die Lagereinrichtung in der Aufnahmeeinrichtung sodann werkzeuglos derart lagerbar ist, dass Lagerkräfte abtragbar sind und insbesondere eine drehmomentenfeste Verbindung vorliegt. Das heißt, dass in der Einbauposition der Lagereinrichtung die Welle des Rührwerks mittels der Antriebseinrichtung antreibbar und somit rotierbar ist, wobei die Lagereinrichtung selbst drehfest montiert ist, sich also nicht gleichlaufend zur Welle des Rührwerks mitdreht, sondern fest steht. Die Lagereinrichtung selbst ist dabei als wellenfeste Wälzlagerung (Lagerpatrone) ausgebildet. Wegen der geneigten Anordnung kann es dabei erforderlich sein, nach oben gerichtete "Abhebekräfte", die sich infolge einer Rührung des Substrats ergeben können, abzutragen. Dies erfolgt typischerweise mittels der Lagerung des oberen Endes des Rührwerks, kann aber zusätzlich auch mittels einer Blockiereinrichtung erreicht werden, die an der Aufnahmeeinrichtung des unteren Endes des Rührwerks angeordnet ist. Ein werkzeugloser "Ausbau" der Lagereinrichtung aus der Aufnahmeeinrichtung und ein sich daran anschließendes Zurückgleiten der Gleitfläche bzw. Zurückrollen der Laufrolle auf der Führungseinrichtung ist dabei vorteilhafterweise ohne Weiteres möglich, sodass eine Wartung oder ein Austausch des Rührwerks ohne eine Beeinträchtigung des Betriebs der Biogasanlage erfolgen kann.

Das erfindungsgemäße Set ermöglicht mithin die folgenden Vorteile:
- Die Deckeneinrichtung des Behälters, insbesondere des Fermenters kann größtenteils, gegebenenfalls sogar vollständig, von einer flexiblen Membran gebildet werden. Ein starrer Deckenteil ist zwecks einer Lagerung des oberen Endes des Rührwerks maximal in einem Randbereich des Behälters notwendig.
- Das Rührwerk ist mit seiner unteren Lagereinrichtung werkzeuglos in dem Behälter fixierbar und in seiner Betriebsposition festlegbar. Ein Austausch des Rührwerks bzw. einzelner Komponenten desselben erfordert demzufolge keinen Einsatz von Werkzeug und somit keine Entleerung und womöglich Reinigung des Behälters.
- Trotz der Nähe des oberen Endes des Rührwerks zur nahesten Wandung des Behälters, möglicherweise sogar der Lagerung des oberen Endes in der Wandung, entfaltet das mindestens eine Rührelement des Rührwerks auch in einem gewissen Abstand von der Wandung des Behälters eine Rührwirkung. Diese ist folglich nicht lokal auf einen Randbereich des Behälters beschränkt.

Bei einem erfindungsgemäßen Set, bei dem das mindestens eine Rührelement des Rührwerks von einem plattenförmigen Paddelelement gebildet ist, das mittels einer Halteeinrichtung drehfest mit der Welle verbunden ist, ist eine Gelenkeinrichtung besonders von Vorteil, mittels derer ein Winkel, der von der Welle und zumindest einem Teilabschnitt der Halteeinrichtung eingeschlossen ist, veränderbar ist. Eine solche Veränderbarkeit der Orientierung des Rührelements relativ zur Welle des Rührwerks ermöglicht einen Ausgleich für eine Schrägstellung des Rührwerks, sofern dies notwendig und/oder erwünscht ist. Insbesondere ist es möglich, das Rührelement soweit "anzuwinkeln", dass es bei einer Umdrehung der Welle nicht aus einer Oberfläche des Substrats heraustritt. Ebenso kann eine Anwinkelung von Vorteil sein, um einen horizontal gemessenen Abstand zwischen einer Anschlussstelle der Halteeinrichtung an der Welle und einem am weitesten von der Welle entfernten Randteil des Rührelements soweit zu reduzieren, dass letzteres nicht Gefahr läuft, gegen die Wandung des Fermenters zu schlagen.

Im Hinblick auf die Rollenlagerung ist es besonders von Vorteil, wenn eine Lauffläche der mindestens einen Laufrolle, mittels derer sie auf einer korrespondierenden Abrollfläche abrollbar ist, konkav oder konvex ausgebildet ist. Eine auf diese Weise ausgebildete Laufrolle ist besonders gut dazu geeignet, mit einer entsprechend ausgeformten Abrollfläche unter Ausbildung eines teilweisen Formschlusses zusammenzuwirken. Beispielsweise ist es gut vorstellbar, dass die Abrollfläche eine konvexe Ausformung aufweist, wobei der konvexe Abschnitt vorteilhafterweise denselben Krümmungsradius aufweist, wie die zugehörige konkav ausgeformte Laufrolle, die sich folglich mit ihrer Oberfläche passend an die Abrollfläche anschmiegt und aufgrund der passenden Ausformung der korrespondierenden Teile seitlich stabilisiert ist. Eine gezielte Führung der Laufrolle ist auf diese Weise besonders einfach möglich.

Ebenso ist es denkbar, dass die Rollenlagerung mehrere Laufrollen aufweist, die jeweils auf einer Abrollfläche abrollen. Ein solches Abrollen kann auch seitlich stattfinden, das heißt eine Kontaktfläche zwischen der Laufrolle und der Abrollfläche kann senkrecht orientiert sein. Jede andere Orientierung, insbesondere eine schräge Orientierung der Kontaktfläche unter einem Winkel von 45°, ist ebenso denkbar.

Ausgehend von einem Behälter, insbesondere Fermenter der eingangs beschriebenen Art wird die zugrunde liegende Aufgabe erfindungsgemäß durch einen Behälter mit den Merkmalen des Anspruchs 4 gelöst. Eine Unterteilung in Deckeneinheit und Membraneinheit ist erfindungsgemäß für ein Rührwerk vorgesehen, dessen oberes Ende an einer Oberseite des Behälters gelagert ist. Bei einer Lagerung des Rührwerks "in der Wandung" des Behälters ist die erfindungsgemäße Unterteilung zwar typischerweise vorhanden, die Platteneinheit jedoch zumindest nicht zur Aufnahme von Lagerkräften vorgesehen. Stattdessen ist die Platteneinheit im Wesentlichen nur zur Montage bzw. Demontage des Rührwerks notwendig. Das heißt, auch in dem Fall der Lagerung des Rührwerks in der Wandung des Behälters ist Deckeneinrichtung in der Regel von zwei separaten Deckeneinheiten gebildet, wobei die Platteneinheit lediglich die Funktion aufweist, eine "Einlassöffnung" für das Rührwerk bereitzustellen, durch die das Rührwerk bei dessen Einbau in den Behälter herabgelassen werden kann. Eine Befestigung des oberen Endes des Rührwerks findet sodann in der Wandung des Behälters statt, beispielsweise in einem in der Wandung eingelassenen Rahmen, in den das obere Ende des Rührwerks von oben her einführbar und an diesem befestigbar ist.

Die erfindungsgemäße Deckeneinrichtung ermöglicht es, die Vorteile der Membraneinheit soweit einzusetzen, wie die Betriebsposition der Rührwerke dies zulässt. Die Platteneinheit wird lediglich benötigt, um das obere Ende des Rührwerks bzw. der Rührwerke zuverlässig zu lagern, das heißt die wirkenden Lagerkräfte abzutragen. Eine solche Lagerung, das heißt die Aufnahme von Lagerkräften, ist mittels der Membraneinheit nicht möglich. Wie vorstehend bereits beschrieben ist, ist die Unterteilung dann besonders sinnvoll, wenn die Platteneinheit möglichst nah an der Wandung des Behälters angeordnet ist. Dies erleichtert den Lastabtrag mittels der Platteneinheit in die Wandungen erheblich.

Dabei ist es besonders von Vorteil, wenn die Platteneinheit parallel zur Bodenplatte ausgerichtet ist. Eine solche Platteneinheit ist vergleichsweise einfach herstellbar.

In einer vorteilhaften Ausgestaltung des erfindungsgemäßen Behälters, insbesondere Fermenters weist die Platteneinheit desselben mindestens eine Montageöffnung auf, durch die hindurch das Rührwerk von einer Außenseite der Platteneinheit her von oben in den Innenraum des Behälters einführbar ist. Eine solche Öffnung ist besonders gut geeignet, um das Rührwerk montieren zu können. Alternativ ist ein "seitliches Andocken" des Rührwerks an die Platteneinheit denkbar. Gleichwohl sind zum einen der Lastabtrag und zum anderen ein Anschluss der Membraneinheit an die Platteneinheit einfacher, wenn das Rührwerk in einer Montageöffnung fixiert ist, die von der Platteneinheit umgeben ist.

Für die Montageöffnung ist es dabei besonders von Vorteil, wenn selbige rechteckig ausgeformt ist, wobei ein freier Querschnitt der Montageöffnung, der nach einem Einsetzen des Rührwerks in den Fermenter verbleibt und den Innenraum des Fermenters mit der Außenseite der Platteneinheit verbindet, im Wesentlichen gasdicht verschließbar ist. Die rechteckige Querschnittsform der Montageöffnung ist insoweit von Vorteil, als sie von ihrer Fläche her so klein wie möglich gehalten werden kann und dennoch genügend Raum für das einzusetzende Rührwerk bietet. Grundsätzlich weist ein Rührwerk typischerweise entlang seiner Welle immer nur ein Rührelement an einer Stelle auf. Das heißt, dass zwei Rührelemente nicht auf "derselben Höhe" der Welle angeordnet sind, das heißt nicht direkt nebeneinander. In einer Draufsicht des Rührwerks betrachtet sind die einzelnen Rührelemente, sofern mehrere von ihnen vorliegen, typischerweise jeweils zu den benachbarten Rührelementen um einen Winkel von 180° versetzt um die Welle des Rührwerks angeordnet. Alternativ ist eine Versetzung um 90° oder jeden beliebigen anderen Winkel denkbar, wenngleich dies eher untypisch ist. Vorteilhafterweise ist die Montageöffnung gerade so groß dimensioniert, dass die Welle nebst einem daran angeschlossenen Rührelement "gerade so" hindurchpasst. Hierbei ist es notwendig, dass das Rührwerk während des Einsetzens in den Fermenter fortlaufend seitlich bewegt wird, um jeweils das nächste Rührelement (sofern vorhanden) derart relativ zu der Montageöffnung zu positionieren, dass es durch die Montageöffnung hindurch passt und nicht mit der Platteneinheit kollidiert. Bei einem Rührwerk, bei dem die Rührelemente um 90° versetzt sind, ist eine reine seitliche Bewegung des Rührwerks beim Einsetzen in den Fermenter nicht ausreichend. Stattdessen muss zusätzlich eine Drehung desselben um die Längsachse der Welle erfolgen, um das jeweils nächste Rührelement relativ zur Montageöffnung auszurichten. Alternativ ist es ebenso denkbar, dass die Montageöffnung Abmessungen aufweist, die ein Einsetzen des Rührwerks erlauben, ohne dass dieses in bestimmte Positionen bewegt werden muss. Allerdings müsste eine solche Montageöffnung deutlich größer dimensioniert werden und folglich die Platteneinheit schwächen. Außerdem fällt eine Abdichtung der Montageöffnung umso schwerer, je größer sie gewählt ist.

Gemäß vorstehend bereits ausgeführter Erläuterungen ist es besonders vorteilhaft, wenn das Rührwerk gegen eine Vertikale geneigt angeordnet ist, wobei die Vertikale und eine Längsachse einer Welle des Rührwerks einen Winkel zwischen 5° und 30° einschließen, wobei zumindest ein Teilbereich der mindestens einen Platteneinheit, der die Montageöffnung aufweist, senkrecht zu der Längsachse der Welle ausgerichtet ist. Die schräge Anordnung ermöglicht es, ein unteres Ende des Rührwerks möglichst weit in Richtung eines Mittelbereichs des Fermenters zu bewegen, dort zu platzieren und gleichzeitig ein oberes Ende möglichst nah an der Wandung des Fermenters zu belassen.

Wie sich bereits aus den vorstehenden Erläuterungen ergibt, ist ein erfindungsgemäßer Behälter dann besonders von Vorteil, wenn ein oberer, von der Bodenplatte des Behälters abgewandter Endabschnitt des Rührwerks mit der Platteneinheit in Kraft übertragender Weise verbunden ist. Die Platteneinheit fungiert als Lager für das obere Ende des Rührwerks und kann entsprechende Lagerkräfte aufnehmen. Zusätzlich ist das Rührwerk mittels seiner Lagereinrichtung an seinem unteren Ende gelagert. Alternativ ist es ebenso denkbar, dass das obere Ende des Rührwerks direkt mit der Wandung des Behälters in Kraft übertragender Weise verbunden ist, also in der Wandung gelagert ist. Dabei wird das obere Ende in einer Betriebsposition des Rührwerks, in dem es fertig montiert ist, "seitlich" aus dem Fermenter heraus geführt, nämlich aus einem Bereich der Wandung des Fermenters. Da - wie vorstehend erläutert - das untere Ende des Rührwerks mittels dessen Lagereinrichtung in Richtung eines Mittelbereichs des Fermenters bewegt ist, ergibt sich eine Schrägstellung des Rührwerks, die im Vergleich zu einer "oberseitigen" Lagerung desselben, das heißt einer Lagerung des oberen Endes in der Deckeneinrichtung, vergrößert ist.

Ausgehend von einem Behälter, insbesondere einem Fermenter der eingangs beschriebenen Art ist die zugrunde liegende Aufgabe grundsätzlich auch durch eine Führungseinrichtung lösbar, mittels derer die Lagereinrichtung des Rührwerks im Zuge eines Einbaus des Rührwerks in den Behälter in Richtung auf die Aufnahmeeinrichtung zu führbar ist, wobei die Aufnahmeeinrichtung an einem Ende der Führungseinrichtung angeordnet ist und eine Einbauposition der Lagereinrichtung festlegt, in der sie eine unbeabsichtigte Bewegung der Lagereinrichtung blockiert. Eine solche Führungseinrichtung ist vorstehend bereits beschrieben worden. Sie ist besonders gut geeignet, die Rollenlagerung des Rührwerks in die Aufnahmeeinrichtung zu führen, wo die Lagereinrichtung des Rührwerks seine Einbauposition einnehmen kann. Erfindungsgemäß vergrößert sich während der Überführung des Rührwerks von der Montageposition in die Einbauposition ein Winkel zwischen der Vertikalen und einer Längsachse der Welle des Rührwerks, so dass das Rührwerk in seiner Betriebsposition, in der es vollständig montiert ist und in Betrieb genommen werden kann, gegen eine Vertikale geneigt angeordnet ist und die Vertikale und eine Längsachse der Welle des Rührwerks einen Winkel zwischen 5° und 60°, vorzugsweise zwischen 10° und 50°, einschließen. Die Neigung ist eine Folge der vorstehend bereits beschriebenen vorteilhaften Lagerung des Rührwerks, dessen unteres Ende möglichst weit von der nahesten Wandung des Behälters entfernt angeordnet ist, während dessen oberes Ende möglichst nah an der nahesten Wandung des Behälters, möglicherweise sogar "in" der Wandung, angeordnet ist. Eine Neigung des Rührwerks in dem erfindungsgemäßen Behälter in der beschriebenen Bandbreite führt bei Höhenmaßen gängiger Behälter zu dem gewünschten Ergebnis.

Grundsätzlich ist der Einbau einer Führungseinrichtung unabhängig von der Konstruktion der Deckeneinrichtung, insbesondere der Frage, ob die Deckeneinrichtung von mindestens zwei unterschiedlichen Deckeneinheiten gebildet ist (Platteneinheit, Membraneinheit). In einer besonders vorteilhaften Ausgestaltung des erfindungsgemäßen Behälters verfügt selbiger gleichwohl über beides, das heißt sowohl eine vorbeschriebene unterteilte Deckeneinrichtung als auch eine vorbeschriebene Führungseinrichtung.

Der erfindungsgemäße Behälter ist dann besonders von Vorteil, wenn die Führungseinrichtung eine lang gestreckte Gleiteinheit oder eine lang gestreckte Abrolleinheit, vorzugsweise ein Laufrohr, aufweist, wobei die Gleiteinheit vorzugsweise glatt ist und die Abrolleinheit vorzugsweise eine konkav oder konvex geformte Abrollfläche aufweist. Der Vorteil einer solchen Ausgestaltung der Führungseinrichtung ist vorstehend bereits erläutert worden.

Die Führungseinrichtung ist dann besonders vorteilhaft, wenn sie zumindest in einem Teil eines Führungsabschnitts einen Neigungswinkel gegenüber einer Horizontalen von mindestens 5°, vorzugsweise mindestens 7,5°, weiter vorzugsweise mindestens 10°, aufweist. Als Führungsabschnitt wird dabei derjenige Abschnitt der Führungseinrichtung bezeichnet, der eine Gleitfläche für die mindestens eine korrespondierende Gleitfläche der Gleitlagerung bzw. eine Abrollfläche für die mindestens eine Laufrolle der Rollenlagerung des Rührwerks umfasst. Eine Neigung zumindest eine Teils des Führungsabschnitts oder vorteilhafterweise des gesamten Führungsabschnitts ist insofern von besonderem Vorteil, als das Rührwerk mit seiner Gleit- bzw. Rollenlagerung besonders einfach auf einer solchen Führungseinrichtung geführt werden kann. Dies liegt darin begründet, dass bei einer schrägen Führungseinrichtung die auf das Rührwerk wirkende Gewichtskraft einen vektoriellen Anteil in Richtung der Führungseinrichtung aufweist, der das Rührwerk letztendlich "von allein" auf der Führungseinrichtung führt. Nach einem Aufsetzen der Gleit- bzw. Rollenlagerung auf die Führungseinrichtung wird sich selbige folglich "von allein" entlang der Führungseinrichtung bewegen. Eine weitere Kraftaufwendung seitens des Monteurs des Rührwerks ist nicht oder nur in geringem Maße erforderlich.

In einer besonders vorteilhaften Ausgestaltung des erfindungsgemäßen Behälters ist die Aufnahmeeinrichtung in einem horizontal, senkrecht zu der mindestens einen Wandung gemessenen Abstand von mindestens 1,5 m, vorzugsweise mindestens 2,0 m, weiter vorzugsweise mindestens 2,5 m, von der Wandung des Behälters entfernt angeordnet. Die Aufnahmeeinrichtung ist dabei typischerweise direkt mit der Bodenplatte des Behälters verbunden. Die Einhaltung des beschriebenen Mindestabstands bedeutet, dass die Lagereinrichtung des Rührwerks in dessen Einbauposition mindestens in einem ebensolchen Abstand zu der Wandung des Behälters eingebaut ist und folglich sichergestellt ist, dass sich eine Rührwirkung der Rührelemente nicht nur in einem Randbereich des Behälters, sondern auch in einem Mittelbereich desselben entfalten kann.

Bezug nehmend auf das eingangs beschriebene Verfahren zum Einbau eines Rührwerks wird die zugrunde liegende Aufgabe erfindungsgemäß durch ein Verfahren mit den Merkmalen des Anspruchs 16 gelöst. Der erfindungsgemäße Verfahrensschritt lautet wie folgt:
c) Während der Überführung des Rührwerks (1, 1') von der Montageposition in die Einbauposition wird ein Winkel zwischen einer vertikalen und einem Längsachse der Welle (12) vergrößert, wobei der vorgenannte Winkel in der Einbauposition Rührwerks (1, 1') vorzugsweise zwischen 5° und 60°, weiter vorzugsweise zwischen 10° und 50°, beträgt.

Dieser Verfahrensschritt ergibt sich bereits aus der vorstehenden Beschreibung und verdeutlicht noch einmal, dass das Rührwerk besonders einfach in einem Randbereich des Behälters eingesetzt werden kann, wobei das Rührwerk in einer Montageposition, das heißt bevor und zu dem Zeitpunkt, wenn die Lagereinrichtung auf die Führungseinrichtung trifft, im wesentlichen senkrecht orientiert ist und erst durch das Abgleiten der Gleitfläche bzw. das Abrollen der Lagerolle auf der Führungseinrichtung, die von der Wandung des Behälters weg führt, eine schräge Stellung einnimmt. Das obere Ende des Rührwerks bleibt währenddessen im Wesentlichen an derselben Position und wird nicht analog zum unteren Ende von der Wandung des Behälters weg bewegt. Insoweit ergibt sich eine Drehung des Rührwerks gegen die Vertikale um einen Drehpunkt, der ungefähr am oberen Ende des Rührwerks angeordnet ist. Das Verfahren ermöglicht es mithin, dass eine Montageöffnung zur Einführung des Rührwerks in das Innere des Behälters lediglich in einem Randbereich desselben vorgesehen werden muss, da eine Bewegung des Rührwerks erst "im Behälter", das heißt mithilfe der Führungseinrichtung stattfindet.

Dabei kann es besonders vorteilhaft sein, das Rührwerk in einem senkrecht zu einer Wandungsfläche einer Wandung des Behälters gemessenen Abstand von maximal 2,0 m von einer Oberseite des Behälters her in selbigen herabzulassen, wobei, nachdem die Lagereinrichtung ihre Einbauposition erreicht hat, ein von der Bodenplatte des Behälters abgewandtes Ende des Rührwerks, das heißt dessen oberes Ende, senkrecht zu der Wandungsfläche der Wandung des Behälters in Richtung eines Mittenbereichs des Behälters bewegt und anschließend fixiert wird. Ein solches Bewegen des oberen Endes des Rührwerks mag in manchen Fällen vorteilhaft sein, sofern ansonsten zu befürchten ist, dass das mindestens eine Rührelement gegen die Wandung des Behälters schlägt. Eine Bewegung des oberen Endes in Richtung des Mittelbereichs des Fermenters findet dabei jedoch nicht in demselben Maße statt, wie dies an dem unteren Ende des Rührwerks der Fall ist. Folglich verbleibt die Welle des Rührwerks auch bei einer solchen Bewegung des oberen Endes des Rührwerks in jedem Fall in einer schrägen Position.

Es versteht sich, dass ein Ausbau des eines jeweiligen Rührwerks genau umgekehrt zu dessen Einbau verlaufen kann. Das heißt, dass die Gleit- bzw. Rollenlagerung mit seiner mindestens einen Gleitfläche bzw. Laufrolle bei einem Ausbau des Rührwerks zunächst die Aufnahmeeinrichtung des Behälters verlässt und sich sodann entlang der Führungseinrichtung in Richtung auf die naheste Wandung des Behälters zu bewegt. Ebenso ist es denkbar, dass das Rührwerk derart an seinem oberen Ende angehoben wird, dass die Lagereinrichtung sich zunächst aus der Aufnahmeeinrichtung löst und sodann "frei schwebend" in dem Substrat bewegt wird, wobei sich aufgrund des Eigengewichts des Rührwerks selbsttätig eine in etwa senkrechte Ausrichtung des Rührwerks einstellt. Im Vergleich zum Einbau des Rührwerks ist dieses Vorgehen vorstellbar, da die Lagereinrichtung nicht in gleicher Weise gezielt an einem bestimmten Ort geführt werden muss. Somit wäre es bei einem Einbau des Rührwerks nur sehr schwierig möglich, die Lagereinrichtung ohne Hilfe der Führungseinrichtung zielsicher in der Aufnahmeeinrichtung zu platzieren.

### Ausführungsbeispiele

Das erfindungsgemäße Set, der erfindungsgemäße Behälter sowie das erfindungsgemäße Verfahren werden nachfolgend anhand eines Ausführungsbeispiels eines in einen Fermenter einzubauenden Rührwerks, das in den Figuren dargestellt ist, näher erläutert. Es zeigt:
- Fig. 1:: Eine Seitenansicht eines in seiner Montageposition befindlichen Rührwerks, das gerade in einen erfindungsgemäßen Behälter in Form eines Fermenters eingesetzt wird,
- Fig. 2:: eine Seitenansicht desselben Rührwerks, jedoch in seiner Betriebsposition,
- Fig. 3:: wie Figur 2, jedoch in einer vereinfachten isometrischen Ansicht,
- Fig. 4:: wie Figur 3, jedoch aus einer anderen Perspektive,
- Fig. 5:: ein Detail einer Rollenlagerung des Rührwerks sowie eine Aufnahmeeinrichtung des Behälters,
- Fig. 6:: eine Seitenansicht eines in dessen Betriebsposition befindlichen weiteren Rührwerks und
- Fig. 7:: Ein Detail einer Einbauöffnung des weiteren Rührwerks.

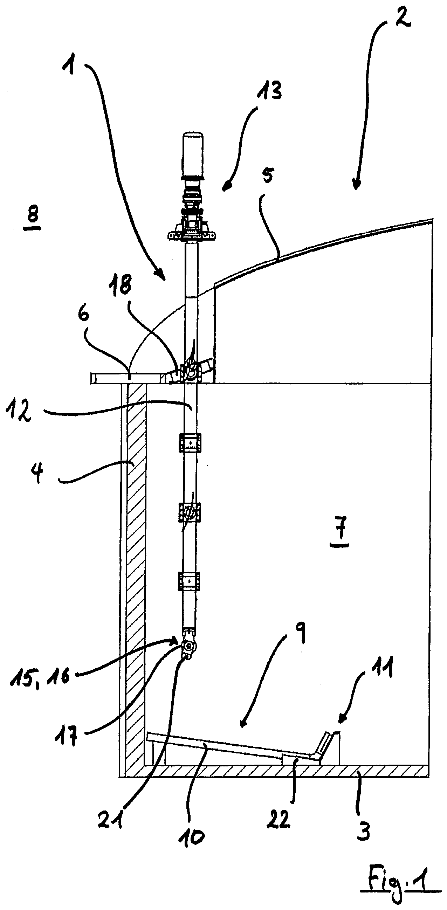

Das Ausführungsbeispiel, das in den Figuren 1 bis 5 dargestellt ist, umfasst sowohl eine Darstellung eines Einbaus eines Rührwerks **1** als auch eine Darstellung von dessen Betriebsposition.

In Figur 1 ist das Rührwerk **1** in seiner Montageposition dargestellt, wobei das Rührwerk **1** gerade in einen erfindungsgemäßen Fermenter **2** eingebaut wird. Der Fermenter **2** umfasst eine Bodenplatte **3,** eine umlaufende Wandung **4** sowie eine zweiteilige Deckeneinrichtung. Diese Deckeneinrichtung ist von zwei Deckeneinheiten, nämlich zum einen von einer Membraneinheit **5** und zum anderen von einer Platteneinheit **6,** gebildet. Die Deckeneinrichtung ist im Wesentlichen gasdicht an die umlaufende Wandung **4** des Fermenters **2** angeschlossen. Unter "im Wesentlichen" ist dabei zu verstehen, dass ein Innenraum **7** des Fermenters **2** derart gegen eine Umgebung **8** des Fermenters **2** abgedichtet ist, dass eine Ansammlung von in dem Fermenter **2** gebildeten Gasen in dem Innenraum **7** stattfinden kann. Ein unkontrollierter Austritt der gebildeten Gase aus dem Innenraum **7** in die Umgebung **8** ist nicht möglich. Gleichwohl mögen gewissen Undichtigkeiten zwischen dem Innenraum **7** und der Umgebung **8** auftreten, die jedoch derart geringfügig sind, dass kein nennenswerter Volumenstrom von dem Innenraum **7** in die Umgebung **8** gelangen kann. Die Membraneinheit **5** und die Platteneinheit **6** sind ebenfalls im Wesentlichen gasdicht miteinander verbunden und bilden auf diese Weise gemeinsam die dichte Deckeneinrichtung.

In dem Fermenter **2** befindet sich eine zu dem Rührwerk **1** gehörige Führungseinrichtung **9,** die mit der Bodenplatte **3** verbunden ist. Diese Führungseinrichtung **9** ist hier von einem lang gestreckten Führungsrohr **10** gebildet, dessen Längsachse in einer Draufsicht des Fermenters **2** betrachtet senkrecht zu der Wandung **2** orientiert ist. Aus der in Figur 1 gezeigten Ansicht wird deutlich, dass das Führungsrohr **10** gegenüber einer Horizontalen einen Winkel von aufweist, der hier ca. 8° beträgt. Eine Orientierung des Führungsrohrs **10** ist dabei so beschaffen, dass ein vertikal gemessener Abstand zwischen der Bodenplatte **3** des Fermenters **2** und dem Führungsrohr **10** mit zunehmendem Abstand von der Wandung **4** des Fermenters **2** abnimmt. An einem der Wandung **4** des Fermenters **2** abgewandten Ende des Führungsrohrs **10** der Führungseinrichtung **9** ist eine gleichfalls zu dem Rührwerk **1** gehörige Aufnahmeeinrichtung **11** angeordnet, deren Funktionalität im Zusammenspiel mit dem Rührwerk **1** weiter unten ausführlich beschreiben ist.

Das Rührwerk **1** verfügt über eine Welle **12,** eine Antriebseinrichtung **13,** vier Rührelemente **14** sowie eine Lagereinrichtung **15.** Die Antriebseinrichtung **13** ist an einem oberen, der Bodenplatte **3** des Fermenters **2** abgewandten Ende der Welle **12** des Rührwerks **1** angeordnet. Die Welle **12** ist um eine Längsachse der Welle **12** verdrehbar zu der Antriebseinrichtung **13** ausgeführt, wobei die Welle mittels der Antriebseinrichtung **13** rotierbar bzw. antreibbar ist. Die Antriebseinrichtung **13** umfasst insbesondere einen Elektromotor sowie ein Getriebe, deren genaue Ausführung bekannt und daher im Rahmen der vorliegenden Anmeldung nicht weiter relevant sind.

An einem der Antriebseinrichtung **13** gegenüberliegenden unteren Ende der Welle **12** des Rührwerks **1** ist die Lagereinrichtung **15** angeordnet. Bei dem Rührwerk **1** ist diese Lagereinrichtung **15** von einem Drehlager gebildet, das als Lagerpatrone in die hohle Welle 12 eingesetzt und somit zusammen mit der Welle 12 aus dem Fermenter 2 entnehmbar ist. An der Lagereinrichtung 15 befindet sich eine Rollenlagerung **16** gebildet, die eine Laufrolle **17** umfasst. Die Lagereinrichtung **15** ist analog zu der Antriebseinrichtung **13** um die Längsachse der Welle **12** relativ zu Welle **12** verdrehbar ausgeführt. Auf diese Weise kann die Lagereinrichtung **15** sowie die Rollenlagerung 16 dazu genutzt werden, das untere Ende des Rührwerks **1** im Zusammenwirken mit der entsprechenden Aufnahmeeinrichtung **11** drehfest zu lagern, hierdurch jedoch eine Rotation der Welle **12** nicht zu blockieren, d. h. dass die Lagereinrichtung 15 sich mit einem äußeren Teil in der Aufnahmeeinrichtung 11 abstützt und somit während der Rotation der Welle 12 still steht, während ein inneres Teil der Lagereinrichtung 15 sich gemeinsam mit der Welle 12 um deren Längsachse dreht.

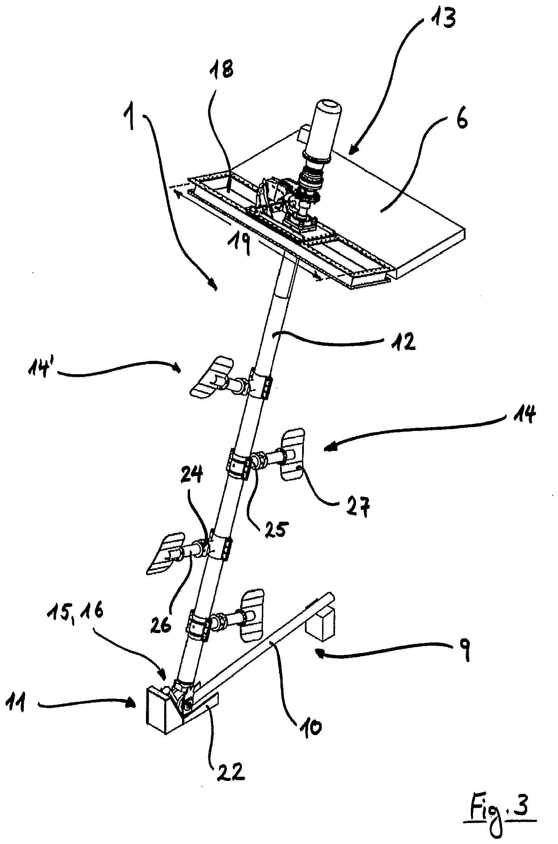

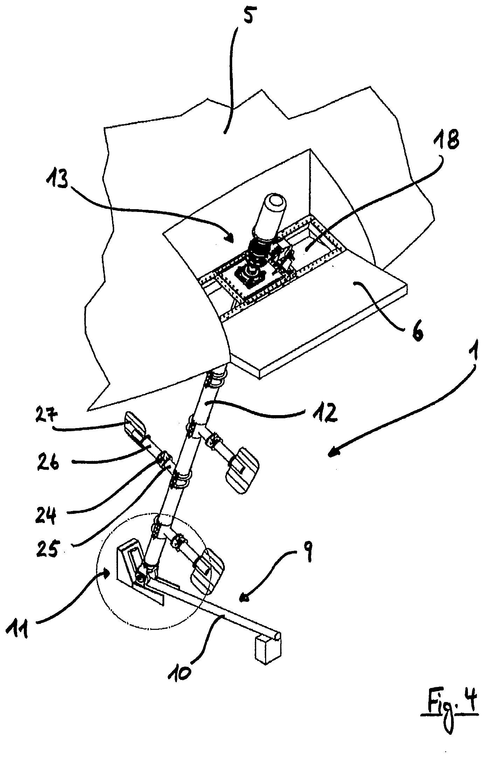

Aus Figur 1 ergibt sich, dass das Rührwerk **1** im Zuge seines Einbaus in den Fermenter **2** durch eine Montageöffnung **18** der Platteneinheit **6** von der Umgebung **8** aus in den Innenraum **7** des Fermenters **2** herabgelassen wird. Die Montageöffnung **18** weist einen rechteckigen Querschnitt auf, wie sich besonders gut aus den Figuren 3 und 4 ergibt. Ein horizontal gemessener Abstand der Montageöffnung **18** von der Wandung **4** des Fermenters **2** beträgt hier ca. 1,0 m. Die Montageöffnung **18** ist in einem Randbereich der Platteneinheit **6** angeordnet, woraus sich implizit die Funktion der Platteneinheit **6** ergibt. Diese erfüllt nämlich gegenüber der Membraneinheit **5** den hauptsächlichen Zweck, dem Rührwerk **1** ein Lager bieten zu können, das heißt Lagerkräfte aufnehmen zu können. Dies wird anhand nachfolgender Erläuterungen verdeutlicht.

Die Geometrie der Montageöffnung **18** ergibt sich besonders gut aus Figur 3. Hier wird ferner ersichtlich, auf welche Weise der Einbau des Rührwerks **1** in den Fermenter **2** erfolgen muss. Eine Breite **19** der Montageöffnung **18** ist nur geringfügig größer gewählt, als eine Strecke **20,** die radial zur Welle **12** gemessen wird und sich ausgehend von einer äußersten Stelle eines Rührelements **14,** die am weitesten von der Welle **12** entfernt ist, bis zu einer äußersten Stelle eines benachbarten Rührelements **14** erstreckt, die ebenfalls am weitesten von der Welle **12** entfernt angeordnet ist. Diese Strecke **20** entspricht gewissermaßen einer "maximalen Breite" des Rührwerks **1.** Mit anderen Worten ist die Montageöffnung **18** gerade so dimensioniert, dass die Welle **12** mitsamt ihren Rührelementen **14** der Breite nach "gerade so" durch die Montageöffnung hindurchpasst, wobei eine Bewegung des Rührwerks **1** im Zuge seines Einbaus in den Fermenter **2** nicht notwendig ist, um die Rührelemente **14** relativ zu der Montageöffnung **18** auszurichten. Alternativ ist es ebenso denkbar, die Montageöffnung schmaler auszuführen, was zur Folge hätte, dass das Rührwerk **1** bei seinem Herablassen in den Innenraum **7** des Fermenters fortlaufend seitlich bzw. rotatorisch bewegt werden müsste, um ein Rührelement **14** nach dem anderen derart relativ zu der Montageöffnung auszurichten, das eine Kollision zwischen der Platteneinheit **6** und dem Rührwerk **1** vermieden wird.

Sofern Rührelemente abweichend von dem hier vorgestellten Ausführungsbeispiel nicht jeweils um 180° versetzt zu einem jeweils benachbarten Rührelement um die jeweilige Welle herum angeordnet sind, sondern beispielsweise um 90° versetzt sind, ist es in aller Regel notwendig, das jeweilige Rührwerk im Zuge eines Ein- bzw. Ausbaus um die Längsachse der jeweiligen Welle zu drehen, um die Rührelemente entsprechend zu der jeweiligen Montageöffnung auszurichten.

Das Rührwerk **1** wird bei seinem Einbau im Wesentlichen senkrecht durch die Montageöffnung **18** hindurch in den Fermenter **2** herabgelassen. Alsbald trifft sodann die Rollenlagerung **16** auf die Führungseinrichtung **9** bzw. deren Führungsrohr **10.** Die Laufrolle **17** und das Führungsrohr **10** sind derart aufeinander abgestimmt, dass die Laufrolle **17** ohne Weiteres auf einer äußeren Mantelfläche des Führungsrohrs **10** abrollen kann. Diese Mantelfläche bildet gewissermaßen die "Abrollfläche" für die Laufrolle **17.** Sobald der Kontakt zwischen der Laufrolle **17** der Rollenlagerung **16** der Lagereinrichtung **15** des Rührwerks **1** und dem Führungsrohr **10** der Führungseinrichtung **9** hergestellt ist, ist es besonders einfach, die Lagereinrichtung **15** entlang des Führungsrohrs **10** zu bewegen bzw. zu "rollen". Die Neigung des Führungsrohrs **10** begünstigt eine solche Bewegung, da bereits die Gewichtskraft des Rührwerks **1** eine Rollbewegung entlang des Führungsrohrs **10** bewirkt. Eine Länge des Führungsrohrs **10** beträgt im gezeigten Beispiel ca. 3,0 m.

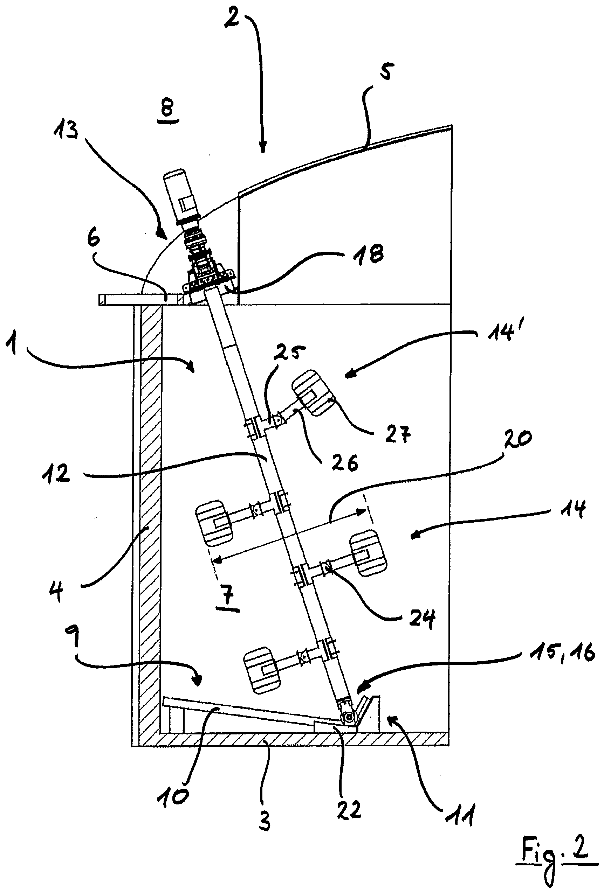

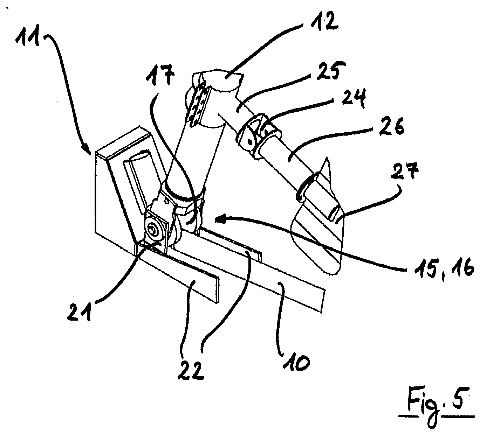

Die Führungseinrichtung **9** ist somit besonders gut geeignet, die Lagereinrichtung **15** mit der Rollenlagerung 16 des Rührwerks **1** aufzunehmen und sodann in Richtung auf die Aufnahmeeinrichtung **11** zu führen. Sobald die Lagereinrichtung **15** in der Aufnahmeeinrichtung **11** angekommen ist, befindet sich die Lagereinrichtung **15** in ihrer Einbauposition. Diese Einbauposition der Lagereinrichtung **15** ist besonders gut in den Figuren 2 bis 5 erkennbar. In der Einbauposition der Lagereinrichtung **15** ist selbige derart in der Aufnahmeeinrichtung **11** positioniert, dass eine Verdrehung der Lagereinrichtung **15** um die Längsachse der Welle **12** des Rührwerks **1** blockiert ist. Dieser Effekt wird im gezeigten Beispiel mittels seitlich an der Laufrolle **17** der Rollenlagerung **16** angeordnete Blockierplatten **21** erzielt, die sich ausgehend von einer Rotationsachse der Laufrolle **17** in Richtung der Bodenplatte **3** des Fermenters **2** erstrecken, wobei eine Länge der Blockierplatten **21** einen Radius der Laufrolle **17** übersteigt, sodass eine Unterkante der Blockierplatten **21** gewissermaßen eine "unterste Stelle" des Rührwerks **1** bilden. Mit anderen Worten würde das Rührwerk **1,** würde man es senkrecht auf eine ebene Fläche herabsenken, zuerst mit den Unterkanten der Blockierplatten **21** die ebene Fläche erreichen. Sobald sich die Lagereinrichtung **15** in ihrer Einbauposition befindet, bilden die Blockierplatten **21** mit korrespondierenden Wandteilen **22** der Aufnahmeeinrichtung **11** einen Formschluss. Dieser Formschluss verhindert letztendlich eine Verdrehung der Lagereinrichtung **15** um die Längsachse der Welle **12** des Rührwerks **1,** auch wenn die Laufrolle 17 mit ihren Spurkränzen von dem Führungsrohr 10 abheben und somit ihren Formschluss verlieren würde. Dieses Zusammenspiel ergibt sich besonders gut aus dem Detail gemäß Figur 5.

Im Übrigen ist die Aufnahmeeinrichtung **11** so ausgebildet, dass die Laufrolle **17** gewissermaßen bis an eine "Anschlagfläche" läuft, die eine weitere Bewegung der Laufrolle **17** in eine von der Wandung **4** des Fermenters **2** weg gerichtete Richtung blockiert. Diese Anschlagfläche wird in dem gezeigten Beispiel technisch dadurch realisiert, dass das Führungsrohr **10** abgewinkelt ist und auf diese Weise eine "tiefste Stelle" ausbildet, in die die Laufrolle **17** einrollt und sich sodann ohne weitere äußere Krafteinwirkung nicht länger bewegen kann. Insbesondere ist eine unbeabsichtigte Bewegung "zurück", das heißt in Richtung der Wandung **4** des Fermenters **2,** nicht möglich, da das Eigengewicht des Rührwerks **1** selbiges in die gebildete tiefste Stelle der Aufnahmeeinrichtung **11** drückt. Auf diese Weise ist das Rührwerk **1** in seiner Position festgelegt, und zwar sowohl was Bewegungen betrifft, die parallel zur Bodenplatte **3** des Fermenters **2** auftreten könnten, als auch hinsichtlich einer Verdrehung der Lagereinrichtung **15.**

Diese Festlegung der Lagereinrichtung **15** in der Aufnahmeeinrichtung **11** ist werkzeuglos möglich. Das führt dazu, dass die beschriebenen Formschlüsse kein weiteres Zutun eines Monteurs oder sonstiger Hilfe bedürfen. Durch diese Konstruktion wird der Vorteil erreicht, dass das Rührwerk **1** besonders einfach installiert und gleichermaßen deinstalliert werden kann, ohne dass hierfür eine Person an dem unteren Ende des Rührwerks **1** tätig werden muss. Dies hat zur Folge, dass das Rührwerk **1** auch im laufenden Betrieb der Biogasanlage, das heißt selbst bei einem mit Substrat gefüllten Fermenter **2,** eingebaut oder ausgebaut werden kann. Insbesondere der Ausbau des Rührwerks **1** ist sehr einfach möglich, da das Rührwerk **1** lediglich in eine Richtung senkrecht nach oben aus der Aufnahmeeinrichtung **11** "herausgezogen" werden muss und sodann durch die Montageöffnung **18** aus dem Fermenter **2** entfernt werden kann.

Bei der Bewegung der Rollenlagerung **16** entlang der Führungseinrichtung **9** verbleibt das obere Ende des Rührwerks **1** im Wesentlichen in dessen voriger Position, wobei die Antriebseinrichtung **13** von dem Innenraum **7** des Fermenters **2** aus betrachtet "oberhalb" der Montageöffnung **18** verbleibt. Da also das obere Ende des Rührwerks **1** seine Position nicht oder nicht wesentlich verändert, gleichwohl jedoch das untere Ende in Form der Lagereinrichtung **15** bewegt wird, ergibt sich eine Schrägstellung des Rührwerks **1.** Diese ist in den Figuren 2 bis **4** besonders gut erkennbar. Die Vorteile dieser Schrägstellung lassen sich am einfachsten unter Heranziehung der Darstellung gemäß Figur 2 erläutern: Aus Figur 2 ergibt sich, dass die Platteneinheit **6** der zweiteiligen Deckeneinrichtung lediglich eine vergleichsweise kurze Ausdehnung in Richtung senkrecht zur Wandung **4** des Fermenters **2** aufweist. Dies ist insoweit besonders vorteilhaft, als ein Kräfteabtrag der an dem oberen Ende des Rührwerks **1** wirkenden Lagerkräfte in die Wandung **4** des Fermenters **2** umso einfacher möglich ist, desto näher die Krafteinleitung des Rührwerks **1** in die Wandung **4** stattfindet. Die Krafteinleitung von Lagerkräften des Rührwerks **1** erfolgt durch eine Befestigung des Rührwerks **1** an der Platteneinheit **6.** Hier kommt insbesondere eine Verschraubung eines um die Welle **12** umlaufenden Flansches mit der Platteneinheit **6** infrage. Die wandungsnahe Montage des oberen Endes der Rührwerks **1** wird schließlich insoweit durch die Schrägstellung desselben begünstigt, als trotz der nahen Anordnung des oberen Endes des Rührwerks **1** an der Wandung **4** die einzelnen Rührelemente **14** in einem gewissen Abstand zu der Wandung **4** angeordnet sind. Dies liegt darin begründet, dass die Rührelemente **14** entlang der Welle **12** verteilt angeordnet sind, wobei aufgrund der Schrägstellung der Welle **12** ein Abstand der Rührelemente **14** von der Wandung **4** umso größer ist, desto näher die Rührelemente **14** zur Bodenplatte **3** des Fermenters **2** angeordnet sind. Dieser gegenüber dem oberen Ende des Rührwerks **1** vergrößerte Abstand ermöglicht es zum einen, das Rührwerk **1** überhaupt in Betrieb zu nehmen. Wäre das Rührwerk **1** ausgehend von seinem oberen Ende senkrecht orientiert, würden die Rührelemente **14** im Betrieb des Rührwerks **1** gegen die Wandung **4** schlagen. Zum anderen ist eine Rührwirkung der Rührelemente **14** aufgrund der Schrägstellung bzw. des vergrößerten Abstands der Rührelemente **14** zur Wandung **4** nicht lediglich auf einen Randbereich des Fermenters **2** beschränkt. Stattdessen wirken die Rührelemente **14** in einer gewissen Entfernung von der Wandung **4,** sodass die Rührwirkung auch in einem Mittelbereich des Fermenters **2** registrierbar ist. Mit anderen Worten ermöglicht die Schrägstellung des Rührwerks **1** die Kombination des Vorteils einer wandungsnahen Lagerung des oberen Endes des Rührwerks **1** mit einer "wandungsfernen" Rührwirkung der Rührelemente **14.** Eine Neigung des Rührwerks **1** gegenüber der Vertikalen beträgt im gezeigten Beispiel ca. 20°. Andere Werte sind jedoch ohne Weiteres denkbar. Dies gilt insbesondere für den Fall, dass das obere Ende eines Rührwerks nicht in einer Platteneinheit, sondern in einer Wandung eines jeweiligen Fermenters gelagert wäre.

Um eine Befestigung des oberen Endes des Rührwerks **1** an der Platteneinheit **6** zu vereinfachen, ist die Platteneinheit **6** im Bereich der Montageöffnung **18** gegen die Horizontale geneigt angeordnet, wie sich besonders gut in den Figuren erkennen lässt. Der Winkel zwischen einer Öffnungsebene der Montageöffnung **18** und der Horizontalen entspricht dabei dem Neigungswinkel der Welle **12** des Rührwerks **1** gegen die Vertikale, beträgt hier folglich ca. 20°.

Die Zweiteilung der Deckeneinrichtung des Fermenters **2** ergibt sich besonders gut aus Figur 4, die ein schräge Ansicht des Rührwerks **1** von einer Außenseite des Fermenters **2** zeigt, wobei die Bauteile des Fermenters **2** selbst nicht dargestellt sind. Erkennbar ist insbesondere die Platteneinheit **6** inklusive der Montageöffnung **18** sowie die Membraneinheit **5.** Die Membraneinheit **5** ist umlaufend gasdicht an die Wandung **4** des Fermenters **2** angeschlossen und nur dort "unterbrochen", das heißt nicht an die Wandung **4** angeschlossen, wo sich eine bzw. die Platteneinheit **6** befindet. An der Stelle, an der die Platteneinheit **6** angeordnet ist, ist die Verbindung zwischen der Membraneinheit **5** und der Wandung **4** unterbrochen und die Membraneinheit **5** stattdessen entlang eines um die Platteneinheit **6** umlaufenden Randes mit selbiger gasdicht verbunden. Die Platteneinheit **6** selbst ist ebenfalls gasdicht. In einem Betriebszustand des Fermenters trifft dies ferner auf die Montageöffnung **18** der Platteneinheit **6** zu, die im Wesentlichen gasdicht verschlossen wird. Auf diese Weise bilden die Platteneinheit **6** und die Membraneinheit **5** gemeinsam die im Wesentlichen gasdichte Deckeneinrichtung des Fermenters **2.**

Der Vorteil dieser Unterteilung liegt insbesondere darin, dass eine Platteneinheit **6** lediglich zu einem vergleichsweise kleinen Anteil zum Einsatz kommt. Dies ist nur deswegen erforderlich, da das Rührwerk **1** an seinem oberem Ende gelagert werden muss. Dies ist jedoch mittels der Membraneinheit **5** nicht möglich, da diese keine Lagerkräfte übertragen kann. Im Übrigen ist die Platteneinheit **6** eher nachteilig, da sie verhältnismäßig aufwendig zu installieren und zu planen ist. Dies gilt insbesondere für Biogasanlagen, deren Fermenter vollständig mittels einer plattenförmigen Deckeneinrichtung verschlossen sind. Derartige Deckeneinrichtungen müssen individuell für jeden Fermenter geplant und aufwendig hergestellt werden. Membraneinheiten sind im Gegensatz dazu deutlich einfacher und schneller zu installieren und bedürfen eines deutlich geringeren Planungsaufwands. Allerdings erlauben sie nicht die Aufnahme von Lagerkräften, weshalb bei Fermentern, die derartige Membraneinheiten verwenden, die jeweiligen Rührwerke von einer Seite des Fermenters durch dessen Wandung in den Innenraum eingeführt und in der Wandung gelagert sind. Dies führt zu den oben bereits ausgeführten Nachteilen. Trotzdem besteht eigentlich grundsätzlich das Bedürfnis, eine Membraneinheit als Deckeneinrichtung zu verwenden. Die erfindungsgemäße Unterteilung der Deckeneinrichtung in mindestens einen Membranteil und mindestens einen Plattenteil löst das Problem des nicht-möglichen Kraftabtrags und kombiniert so die Vorteile der jeweiligen Komponenten.

Eine weitere Besonderheit des Rührwerks **1** besteht in der Anbindung der Rührelemente **14** an die Welle **12.** Die Rührelemente **14** sind hier in Form von rechteckigen Paddelelementen **27** gebildet. Diese sind mittels Halteeinrichtungen **23** mit der Welle **12** kraftschlüssig verbunden. Die Halteeinrichtungen **23** weisen jeweils eine Gelenkeinrichtung **24** auf. Mittels einer solchen Gelenkeinrichtung **24** ist es möglich, einen Anstellwinkel des jeweiligen Paddelelements **27** relativ zu der Welle **12** zu verändern. Dies ist besonders gut bei dem obersten Rührelement **14'** in Figur 2 erkennbar. Die Gelenkeinrichtung **24** ist hier entlang der Halteeinrichtung **23** angeordnet und ist und standardmäßig so ausgerichtet, dass ein der Welle **12** zugewandter Teil **25** sowie ein der Welle **12** abgewandter Teil **26** der Halteeinrichtung **23** parallel ausgerichtet sind. Eine Verdrehung der beiden Teile **25, 26** gegeneinander ist mittels der Gelenkeinrichtung **24** möglich. Dies hat insbesondere den Effekt, dass sich eine senkrecht zur Längsachse der Welle **12** gemessene "Länge" der Halteeinrichtung **23** reduziert, umso stärker die beiden Teile **25, 26** der Halteeinrichtung **23** gegeneinander verdreht sind. Im gezeigten Beispiel wird dieser Effekt genutzt, um sicherzustellen, dass das oberste Rührelement **14** bei seiner Rotation um die Längsachse der Welle **12** nicht versehentlich gegen die Wandung **4** des Fermenters **2** prallt. Je nachdem, wie stark die Welle **12** gegen die Vertikale geneigt ist und in welcher Höhe die Rührelemente **14** entlang der Welle **12** angeordnet sind, kann eine solche Anwinkelung der Halteeinrichtung **23** notwendig sein, um eine Kollision des jeweiligen Paddelelements mit einer jeweiligen Wandung zu vermeiden. Außerdem kann eine Veränderung der Ausrichtung der Paddelelemente **27** relativ zur Welle **12** sinnvoll sein, um sicherzustellen, dass das Paddelelemente **27** im Laufe einer Umdrehung um die Längsachse der Welle **12** nicht aus dem in dem Fermenter **2** befindlichen Substrat austritt.

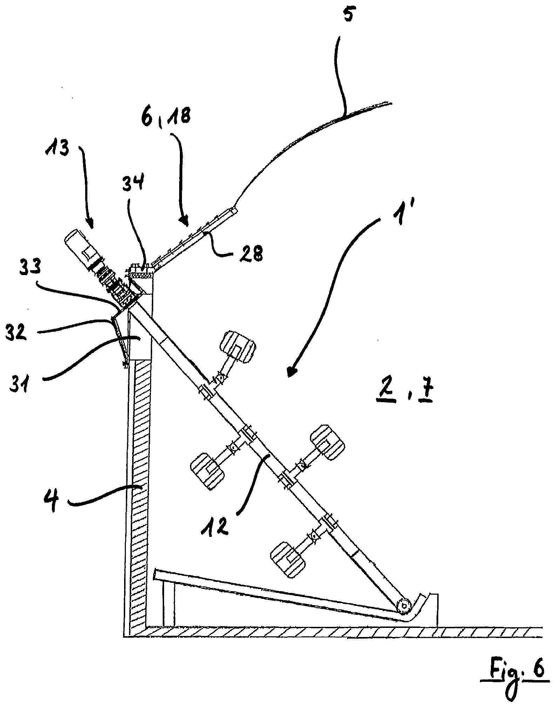

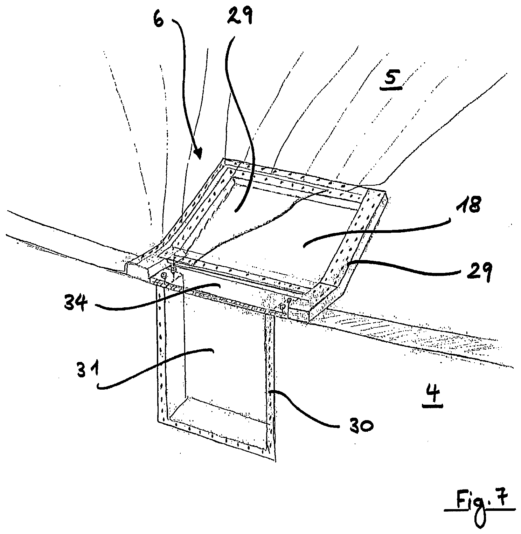

In einem weiteren Ausführungsbeispiel, das in Figur 6 dargestellt ist, ist ein Rührwerk **1'** derart in dem Fermenter **2** montiert, dass die Welle **12** des Rührwerks **1'** die Wandung **4** durchstößt. Ein Detail einer entsprechenden Einbauöffnung ist Figur 7 entnehmbar. Im Unterschied zum Rührwerk **1** ist das alternative Rührwerk **1'** schräger gegenüber einer Vertikalen angeordnet. Ein Neigungswinkel der Welle **12** beträgt hier ca. **50°.** Im Übrigen verläuft der Einbauvorgang des Rührwerks **1'** identisch zu demjenigen des Rührwerks **1.** So erklärt es sich auch, dass eine Deckeneinrichtung des dargestellten Fermenters **2** auch hier von einer Art Platteneinheit **6'** und im Übrigen von einer Membraneinheit **5** gebildet ist. Die Platteneinheit **6'** wird dabei allerdings nicht dazu verwendet, das obere Ende des Rührwerks **1'** zu lagern und Lagerkräfte abzutragen, sondern dient ausschließlich zu Montage- bzw. Demontagezwecken. Daher ist die Deckeneinheit **6'** ausschließlich von einer Montageöffnung **18** gebildet, durch die Rührwerk **1'** in den Fermenter **2** hinab gelassen bzw. aus diesem heraus gehoben werden kann. Die Montageöffnung **18** ist besonders gut aus Figur 7 erkennbar. Sie umfasst einen starren Rahmen **28,** der mittels einer Abdeckung **29** im Wesentlichen gasdicht verschließbar ist, wobei in Figur 7 die Abdeckung **29** exemplarisch lediglich teilweise dargestellt ist. Die Membraneinheit **5** ist dichtend an den Rahmen **28** angeschlossen, so dass an einem Übergang von der Platteneinheit **6'** zur Membraneinheit **5** kein Gas aus dem Innenraum **7** des Fermenters **2** entweichen kann.

Nach einem Herablassen des Rührwerks **1'** durch die Montageöffnung **18** in den Fermenter **2** wird die Lagereinrichtung **15** ausgehend von einer zunächst senkrechten Montageposition (das gesamte Rührwerk hängt dabei an einer Hebevorrichtung) mittels der an der Lagereinrichtung 15 befindlichen Rollenlagerung **16,** die die Laufrolle **17** umfasst, in deren Einbauposition bewegt. Dieser Verfahrensschritt ist identisch zum Einbau des vorstehend beschriebenen Rührwerks **1.** Anschließend wird das oberes Ende des Rührwerks **1'** seitlich aus der Montageöffnung **18** heraus in einen Wandrahmen **30** bewegt, in dem es letztendlich befestigt wird. Der Wandrahmen **30** bildet eine Lageröffnung **31,** in der das Rührwerk **1'** befestigbar ist, sodass Lagerkräfte abgetragen und von der Wandung **4** des Fermenters **2** aufnehmbar sind. Eine Übergang von der Montageöffnung **18** in die Lageröffnung **31** ist während der Montage des Rührwerks **1'** frei. Sobald das obere Ende des Rührwerks **1'** in der Lageröffnung **31** befestigt ist, befindet sich das Rührwerk **1'** in seiner Betriebsposition. Die Befestigung erfolgt mittels Fixierplatten **32,** die gleichermaßen mit dem Wandrahmen **30** als auch mit einer Lagerplatte **33** der Rührwerks **1'** verbunden sind, wobei die Lagerplatte **33** direkt unterhalb der Antriebseinrichtung **13** des Rührwerks **1'** angeordnet ist. Sobald sich das Rührwerk **1'** in seiner Betriebsposition befindet, werden sowohl die Montageöffnung **18** als auch die Lageröffnung **31** im Wesentlichen gasdicht verschlossen. Ein Übergang zwischen der Montageöffnung **18** und der Lageröffnung **31** wird mittels einer Planke **34** verschlossen, die auf gegenüberliegende Seiten der Wandung **4** geschraubt ist. Dies ergibt sich besonders gut aus Figur 7.

Die Art der Befestigung des Rührwerks **1'** in der Wandung **4** hat den Vorteil, dass die Deckeneinrichtung im Betrieb des Fermenters **2** frei von Rührwerken ist. Der benötigte Platz für die Montageöffnung **18** ist vergleichsweise gering, so dass ein umso größerer Teil der Deckeneinrichtung von der Membraneinheit **5** gebildet wird. Der Fermenter **2** weist hier eine Höhe von ca. 10 m auf.

### Bezugszeichenliste

- 1, 1': Rührwerk
- 2: Fermenter
- 3: Bodenplatte
- 4: Wandung
- 5: Membraneinheit
- 6, 6': Platteneinheit
- 7: Innenraum
- 8: Umgebung
- 9: Führungseinrichtung
- 10: Führungsrohr
- 11: Aufnahmeeinrichtung
- 12: Welle
- 13: Antriebseinrichtung
- 14, 14': Rührelement
- 15: Lagereinrichtung
- 16: Rollenlagerung
- 17: Laufrolle
- 18: Montageöffnung
- 19: Breite
- 20: Strecke
- 21: Blockierplatte
- 22: Wandteil
- 23: Halteeinrichtung
- 24: Gelenkeinrichtung
- 25: Teil
- 26: Teil
- 27: Paddelelement
- 28: Rahmen
- 29: Abdeckung
- 30: Wandrahmen
- 31: Lageröffnung
- 32: Fixierplatte
- 33: Lagerplatte
- 34: Planke

## Patentansprüche

1. Set umfassend einen Behälter (2) und ein Rührwerk (1, 1'), das Rührwerk (1, 1') aufweisend eine rotierbare Welle (12), eine Antriebseinrichtung (13), mittels derer die Welle (12) antreibbar ist, sowie mindestens ein an der Welle (12) angeordnetes Rührelement (14, 14'), das gemeinsam mit der Welle (12) rotierbar ist, wobei die Welle (12) eine damit verbundene Lagereinrichtung (15) aufweist, die in einem der Antriebseinrichtung (13) abgewandten Endbereich der Welle (12) angeordnet ist, wobei die Welle (12) um ihre Längsachse relativ zu der Lagereinrichtung (15) verdrehbar ist, wobei mittels der Lagereinrichtung (15) das Rührwerk (1, 1') in einer mit dem Behälter (2) verbundenen Aufnahmeeinrichtung (11) lagerbar ist, wobei die Lagereinrichtung (15) mittels einer Gleitlagerung oder einer Rollenlagerung (16) entlang einer Führungseinrichtung (9) in die Aufnahmeeinrichtung (11) bewegbar ist, wobei die Gleitlagerung mindestens eine Gleitfläche umfasst, die auf einer korrespondierenden Gleitfläche der Führungseinrichtung (9) bewegbar ist, oder die Rollenlagerung (16) mindestens eine Laufrolle (17) umfasst, die auf einer Abrollfläche der Führungseinrichtung (9) rollbar ist, **dadurch gekennzeichnet, dass** die Welle (12) mittels der Lagereinrichtung (15) derart entlang der Führungseinrichtung (9) bewegbar ist, dass das Rührwerk (1, 1') zwischen einer Montageposition und einer Einbauposition überführbar ist, wobei ein Winkel zwischen der Längsachse der Welle (12) und einer Vertikalen während der Überführung des Rührwerks (1, 1') von der Montageposition in die Einbauposition vergrößerbar ist.

2. Set nach Anspruch (1), **dadurch gekennzeichnet, dass** der Winkel zwischen der Längsachse der Welle (12) und der Vertikalen bei Vorliegen des Rührwerks (1, 1') in seiner Einbauposition zwischen 5° und 60°, vorzugsweise zwischen 10° und 50°, beträgt.

3. Set nach Anspruch 1 oder 2, wobei das mindestens eine Rührelement (14, 14') von einem plattenförmigen Paddelelement (27) gebildet ist, das mittels einer Halteeinrichtung (23) drehfest mit der Welle (12) verbunden ist, **gekennzeichnet durch** eine Gelenkeinrichtung (24), mittels derer ein Winkel, der von der Welle (12) und zumindest einem Teilabschnitt der Halteeinrichtung (23) eingeschlossen ist, veränderbar ist.

4. Behälter, insbesondere Fermenter (2) zur Fermentierung biologisch zersetzbarer Stoffe, aufweisend mindestens eine Bodenplatte (3), mindestens eine Wandung (4) sowie mindestens eine Deckeneinrichtung, wobei mittels der Bodenplatte (3), der Wandung (4) und der Deckeneinrichtung ein Innenraum (7) des Behälters (2) im Wesentlich gasdicht eingefasst ist, wobei der Behälter (2) mindestens ein Rührwerk (1, 1') aufweist, mittels dessen in dem Innenraum (7) des Behälters (2) befindliche Stoffe rührbar sind, wobei die Deckeneinrichtung von mindestens zwei Deckeneinheiten, nämlich mindestens einer Platteneinheit (6, 6') und mindestens einer Membraneinheit (5), gebildet ist, wobei die Platteneinheit (6, 6') von einem plattenförmigen Bauteil und die Membraneinheit (5) von einer Membran gebildet ist, wobei die Platteneinheit (6, 6') und die Membraneinheit (5) in Bereichen, in denen sie aneinander stoßen, im Wesentlichen gasdicht miteinander verbunden sind, wobei der Behälter mindestens eine Führungseinrichtung (9) aufweist, mittels derer die Lagereinrichtung (15) des Rührwerks (1, 1') im Zuge einer Überführung des Rührwerks (1, 1') von einer Montageposition in eine Einbauposition in Richtung auf eine Aufnahmeeinrichtung (11) zu führbar ist, wobei die Aufnahmeeinrichtung (11) an einem Ende der Führungseinrichtung (9) angeordnet ist und die Einbauposition festlegt, in der sie eine unbeabsichtigte Bewegung der Lagereinrichtung (15) blockiert, **dadurch gekennzeichnet, dass** das Rührwerk (1, 1') in seiner Einbauposition gegen eine Vertikale geneigt angeordnet ist, wobei die Vertikale und eine Längsachse einer Welle (12) des Rührwerks (1, 1') einen Winkel zwischen 5° und 60° einschließen.

5. Behälter (2) nach Anspruch 4, **dadurch gekennzeichnet, dass** zumindest ein Teilbereich der mindestens einen Platteneinheit (6, 6'), der die Montageöffnung (18) aufweist, senkrecht zu der Längsachse der Welle (12) ausgerichtet ist.

6. Behälter (2) nach Anspruch 4 oder 5, **dadurch gekennzeichnet, dass** die Platteneinheit (6, 6') parallel zur Bodenplatte (3) ausgerichtet ist.

7. Behälter (2) nach einem der Ansprüche 4 bis 6, **dadurch gekennzeichnet, dass** die Platteneinheit (6, 6') mindestens eine Montageöffnung (18) aufweist, durch die hindurch das Rührwerk (1, 1') von einer Umgebung (8) der Platteneinheit (6) her in den Innenraum (7) des Fermenters (2) einführbar ist.

8. Behälter (2) nach Anspruch 7, **dadurch gekennzeichnet, dass** die Montageöffnung (18) rechteckig ausgeformt ist, wobei ein freier Querschnitt der Montageöffnung (18), der nach einem Einsetzen des Rührwerks (1, 1') in den Fermenter (2) verbleibt und den Innenraum (7) des Fermenters (2) mit der Außenseite der Platteneinheit (6, 6') verbindet, im Wesentlichen gasdicht verschließbar ist.

9. Behälter (2) nach Anspruch 7 oder 8, **dadurch gekennzeichnet, dass** die Montageöffnung (18) in einem horizontal gemessenen Abstand von weniger als 2,0 m, vorzugsweise weniger als 1,5 m, weiter vorzugsweise weniger als 1,0 m, von der Wandung (4) des Fermenters (2) entfernt angeordnet ist.

10. Behälter (2) nach den Ansprüchen 4 bis 9, **dadurch gekennzeichnet, dass** ein oberer, von der Bodenplatte (3) des Fermenters (2) abgewandter Endabschnitt des Rührwerks (1) mit der Platteneinheit (6) in Kraft übertragender Weise verbunden ist.

11. Behälter (2) nach Anspruch 4, aufweisend eine mit der Bodenplatte (3) verbundene Aufnahmeeinrichtung (11), mittels derer eine Lagereinrichtung (15) des Rührwerks (1, 1'), die in einem der Bodenplatte (3) des Behälters (2) zugewandten Endbereich einer Welle (12) des Rührwerks (1, 1') angeordnet ist, aufnehmbar ist, sowie eine Führungseinrichtung (9), mittels derer die Lagereinrichtung (15) des Rührwerks (1, 1') im Zuge einer Überführung des Rührwerks (1, 1') von einer Montageposition in eine Einbauposition in Richtung auf die Aufnahmeeinrichtung (11) zu führbar ist, wobei die Aufnahmeeinrichtung (11) an einem Ende der Führungseinrichtung (9) angeordnet ist und die Einbauposition festlegt, in der sie eine unbeabsichtigte Bewegung der Lagereinrichtung (15) blockiert, **dadurch gekennzeichnet, dass** sich ein Winkel zwischen der Vertikalen und einer Längsachse der Welle des Rührwerks (1, 1') während der Überführung von der Montageposition in die Einbauposition vergrößert, so dass das Rührwerk (1) in dessen Einbauposition gegen die Vertikale geneigt angeordnet ist.

12. Behälter nach Anspruch 11, **dadurch gekennzeichnet, dass** die Vertikale und eine Längsachse der Welle des Rührwerks (1) einen Winkel zwischen 5° und 60°, vorzugsweise zwischen 10° und 50°, einschließen.

13. Behälter nach Anspruch 11 oder 12, **dadurch gekennzeichnet, dass** die Führungseinrichtung (9) eine lang gestreckte Gleiteinheit oder eine lang gestreckte Abrolleinheit, vorzugsweise ein Führungsrohr (10), aufweist, wobei die Gleiteinheit vorzugsweise glatt ausgebildet ist und die Abrolleinheit vorzugsweise eine im Querschnitt konkav oder konvex geformte Abrollfläche aufweist.

14. Behälter (2) nach einem der Ansprüche 11 bis 13, **dadurch gekennzeichnet, dass** die Führungseinrichtung (9) in einem Führungsabschnitt einen Neigungswinkel gegenüber einer Horizontalen von mindestens 5°, vorzugsweise mindestens 7,5°, weiter vorzugsweise mindestens 10°, aufweist.

15. Behälter (2) nach einem der Ansprüche 11 bis 14, **dadurch gekennzeichnet, dass** die Aufnahmeeinrichtung (11) in einem horizontal, senkrecht zu der mindestens einen Wandung gemessenen Abstand von mindestens 1,5 m, vorzugsweise mindestens 2,0 m, weiter vorzugsweise mindestens 2,5 m, von der Wandung (4) entfernt angeordnet ist.

16. Verfahren zum Einbau eines Rührwerks (1, 1') in einen Behälter, insbesondere in einen Fermenter (2), umfassend die folgenden Verfahrensschritte:
a) Eine Lagereinrichtung (15) des Rührwerks (1, 1'), die an einem einer Bodenplatte (3) des Behälters (2) zugewandten Ende einer Welle (12) des Rührwerks (1, 1') angeordnet und mit dieser verbunden ist, wird in einer mit der Bodenplatte (3) des Behälters (2) verbundenen Aufnahmeeinrichtung (11) des Behälters (2) gelagert, wobei, um die Lagereinrichtung (15) des Rührwerks (1, 1') in die Aufnahmeeinrichtung (11) einzusetzen, die Lagereinrichtung (15) ausgehend von einer Montageposition des Rührwerks (1, 1') vorerst in eine Führungseinrichtung (9) eingesetzt und mittels dieser Führungseinrichtung (9) in Richtung auf die Aufnahmeeinrichtung (11), die an einem Ende der Führungseinrichtung (9) angeordnet ist, zu geführt wird, bis die Lagereinrichtung (15) die Aufnahmeeinrichtung (11) erreicht, von dieser aufgenommen wird und somit das Rührwerk (1, 1') seine Einbauposition einnimmt.
b) Das Rührwerk (1, 1') wird in seinem oberen, der Bodenplatte (3) abgewandten Lagerabschnitt gelagert,
**gekennzeichnet durch** den folgenden Verfahrensschritt:
c) Während der Überführung des Rührwerks (1, 1') von der Montageposition in die Einbauposition wird ein Winkel zwischen einer Vertikalen und einer Längsachse der Welle (12) vergrößert..

17. Verfahren nach Anspruch 16, **dadurch gekennzeichnet, dass** das Rührwerk (1, 1') in einem senkrecht zu einer Wandungsfläche einer Wandung (4) des Behälters (2) gemessenen Abstand von maximal 2,0 m von einer Oberseite des Behälters (2) her in selbigen vorzugsweise vertikal herabgelassen wird, wobei, nachdem die Lagereinrichtung (15) ihre Einbauposition erreicht hat, ein von der Bodenplatte (3) des Behälters (2) abgewandtes Ende des Rührwerks (1, 1') senkrecht zu der Wandungsfläche der Wandung (4) des Behälters (2) in Richtung eines Mittenbereichs des Behälters (2) bewegt und anschließend fixiert wird.

18. Verfahren nach Anspruch 16 oder 17, **dadurch gekennzeichnet, dass** das Rührwerk (1') durch eine Montageöffnung (18), die sich in einer Deckeneinrichtung des Behälters (2) befindet, in den Behälter (2) herabgelassen wird und anschließend in einer Lageröffnung (31), die sich in einer Wandung (4) des Behälters (2) befindet fixiert wird, sodass das Rührwerk (1') seine Betriebsposition einnimmt.

## Claims

1. A set, comprising a container (2) and an agitator (1, 1'), the agitator (1, 1') having a rotatable shaft (12), a drive device (13) by means of which the shaft (12) can be driven, and at least one agitator element (14, 14') arranged on the shaft (12), which can be rotated together with the shaft (12), wherein the shaft (12) has a bearing device (15) which is connected thereto and is arranged in an end region of the shaft (12) facing away from the drive device (13), wherein the shaft (12) is rotatable about its longitudinal axis relative to the bearing device (15), wherein the agitator (1, 1') can be supported by means of the bearing device (15) in a receiving device (11) connected to the container (2), wherein the bearing device (15) can be moved along a guide device (9) into the receiving device (11) by means of a slide bearing or a roller bearing (16), wherein the slide bearing comprises at least one slide surface which can be moved on a corresponding slide surface of the guide device (9), or the roller bearing (16) comprises at least one running roller (17) which can be rolled on a rolling surface of the guide device (9), **characterized in that** the shaft (12) can be moved along the guide device (9) by means of the bearing device (15) in such a manner that the agitator (1, 1') can be transferred between a mounting position and an installation position, wherein an angle between the longitudinal axis of the shaft (12) and a vertical can be increased during the transfer of the agitator (1, 1') from the mounting position into the installation position.

2. The set according to claim 1, **characterized in that** the angle between the longitudinal axis of the shaft (12) and the vertical is between 5° and 60°, preferably between 10° and 50°, when the agitator (1, 1') is in its installation position.

3. The set according to claim 1 or 2, wherein the at least one agitator element (14, 14') is formed by a plate-shaped paddle element (27) which is connected to the shaft (12) in a rotationally fixed manner by means of a holding device (23), **characterized by** an articulation device (24) by means of which an angle which is enclosed by the shaft (12) and at least one partial section of the holding device (23) can be varied.

4. A container, in particular fermenter (2), for fermenting biologically decomposable substances, having at least one base plate (3), at least one wall (4) and at least one ceiling device, wherein an interior space (7) of the container (2) is substantially enclosed in a gas-tight manner by means of the base plate (3), the wall (4) and the ceiling device, wherein the container (2) has at least one agitator (1, 1'), by means of which substances located in the interior space (7) of the container (2) can be agitated, wherein the ceiling device is formed by at least two ceiling units, namely at least one plate unit (6, 6') and at least one membrane unit (5), wherein the plate unit (6, 6') is formed by a plate-shaped component and the membrane unit (5) is formed by a membrane, wherein the plate unit (6, 6') and the membrane unit (5) are connected to one another in a substantially gas-tight manner in regions in which they rest against each other, wherein the container has at least one guide device (9) by means of which the bearing device (15) of the agitator (1, 1') can be fed in the course of a transfer of the agitator (1, 1') from a mounting position into an installation position towards a receiving device (11), wherein the receiving device (11) is arranged at an end of the guide device (9) and defines the installation position in which it blocks unintentional movement of the bearing device (15), **characterized in that** the agitator (1, 1') in its mounting position is arranged inclined against a vertical, wherein the vertical and a longitudinal axis of a shaft (12) of the agitator (1, 1') enclose an angle between 5° and 60°.

5. The container (2) according to claim 4, **characterized in that** at least one partial region of the at least one plate unit (6, 6') which includes the mounting opening (18) is oriented perpendicular to the longitudinal axis of the shaft (12).

6. The container (2) according to claim 4 or 5, **characterized in that** the plate unit (6, 6') is oriented parallel to the base plate (3).

7. The container (2) according to any one of claims 4 to 6, **characterized in that** the plate unit (6, 6') has at least one mounting opening (18) through which the agitator (1, 1') can be inserted coming from a surrounding area (8) of the plate unit (6) into the interior space (7) of the fermenter (2).

8. The container (2) according to claim 7, **characterized in that** the mounting opening (18) is of rectangular shape, wherein a free cross-section of the mounting opening (18), which remains after insertion of the agitator (1, 1') into the fermenter (2) and connects the interior space (7) of the fermenter (2) to the outside of the plate unit (6, 6'), can be closed in a substantially gas-tight manner.

9. The container (2) according to claim 7 or 8, **characterized in that** the mounting opening (18) is arranged spaced apart from the wall (4) of the fermenter (2) at a horizontally measured distance of less than 2.0 m, preferably less than 1.5 m, more preferably less than 1.0 m.

10. The container (2) according to claims 4 to 9, **characterized in that** an upper end section of the agitator (1) facing away from the base plate (3) of the fermenter (2) is connected to the plate unit (6) in a force transmitting manner.

11. The container (2) according to claim 4, having a receiving device (11) which is connected to the base plate (3) and by means of which a bearing device (15) of the agitator (1, 1'), which is arranged in an end region of a shaft (12) of the agitator (1, 1') facing the base plate (3) of the container (2), can be received, and having a guide device (9), by means of which the bearing device (15) of the agitator (1, 1') can be fed in the course of a transfer of the agitator (1, 1') from a mounting position into an installation position towards the receiving device (11), wherein the receiving device (11) is arranged at an end of the guide device (9) and defines the installation position in which it blocks an unintentional movement of the bearing device (15), **characterized in that** an angle between the vertical and a longitudinal axis of the shaft of the agitator (1, 1') increases during the transfer from the mounting position to the installation position, so that the agitator (1) in its installation position is arranged inclined against to the vertical.

12. The container according to claim 11, **characterized in that** the vertical and a longitudinal axis of the shaft (12) of the agitator (1) enclose an angle between 5° and 60°, preferably between 10° and 50°.

13. The container according to claim 11 or 12, **characterized in that** the guide device (9) has an elongate sliding unit or an elongate rolling unit, preferably a guide tube (10), wherein the sliding unit is preferably formed smooth and the rolling unit preferably has a rolling surface that is formed concave or convex in cross-section.

14. The container (2) according to any one of claims 11 to 13, **characterized in that** in a guide section, the guide device (9) has an angle of inclination with respect to a horizontal of at least 5°, preferably at least 7.5°, more preferably at least 10°.

15. The container (2) according to any one of claims 11 to 14, **characterized in that** the receiving device (11) is arranged spaced apart from the wall (4) at a horizontally measured perpendicular distance of at least 1.5 m, preferably at least 2.0 m, more preferably at least 2.5 m.

16. A method for installing an agitator (1, 1') in a container, in particular a fermenter (2), comprising the following method steps:
a) a bearing device (15) of the agitator (1, 1'), which is arranged at an end of a shaft (12) of the agitator (1, 1') facing a base plate (3) of the container (2) and is connected thereto, is mounted in a receiving device (11) of the container (2) connected to the base plate (3) of the container (2), wherein, in order to insert the bearing device (15) of the agitator (1, 1') into the receiving device (11), the bearing device (15), starting from a mounting position of the agitator (1, 1'), is first inserted into a guide device (9) and is fed by means of this guide device (9) towards the receiving device (11), which is arranged at an end of the guide device (9), until the bearing device (15) reaches the receiving device (11), is received by the same and thus the agitator (1, 1') assumes its installation position.
b) The agitator (1, 1') is mounted in its upper bearing section facing away from the base plate (3),
**characterized by** the following method step:
c) During the transfer of the agitator (1, 1') from the mounting position to the installation position, an angle between a vertical and a longitudinal axis of the shaft (12) is increased.

17. The method according to claim 16, **characterized in that** the agitator (1, 1'), at a distance of maximally 2.0 m measured perpendicular to a wall surface of a wall (4) of the container (2), is lowered, preferably vertically, from an upper side of the container (2) into the same, wherein, after the bearing device (15) has reached its installation position, an end of the agitator (1, 1') facing away from the base plate (3) of the container (2) is moved perpendicular to the wall surface of the wall (4) of the container (2) in the direction of a central region of the container (2) and is subsequently fixed.

18. The method according to claim 16 or 17, **characterized in that** the agitator (1') is lowered into the container (2) through a mounting opening (18) located in a ceiling device of the container (2) and is subsequently fixed in a bearing opening (31) located in a wall (4) of the container (2) so that the agitator (1') assumes its operating position.

## Revendications

1. Set comprenant un récipient (2) et un mélangeur (1, 1'), le mélangeur (1, 1') présentant un arbre rotatif (12), un dispositif d'entraînement (13) au moyen duquel l'arbre (12) peut être entraîné, ainsi qu'au moins un élément de mélange (14, 14') disposé sur l'arbre (12) qui peut tourner conjointement avec l'arbre (12), dans lequel l'arbre (12) présente un dispositif de palier (15) relié à celui-ci qui est disposé dans une partie d'extrémité de l'arbre (12) détournée du dispositif d'entraînement (13), dans lequel l'arbre (12) peut tourner sur son axe longitudinal par rapport au dispositif de palier (15), dans lequel, au moyen du dispositif de palier (15), le mélangeur (1, 1') peut être disposé dans un dispositif de réception (11) relié au récipient (2), dans lequel le dispositif de palier (15) est mobile dans le dispositif de réception (11) au moyen d'un palier de coulissement ou d'un palier à rouleaux (16) le long d'un dispositif de guidage (9), dans lequel le palier de coulissement comprend au moins une face de coulissement qui est mobile sur une face de coulissement correspondante du dispositif de guidage (9), ou bien le palier à rouleaux (16) comprend au moins un galet de roulement (17) qui peut rouler sur une face de déroulement du dispositif de guidage (9), **caractérisé en ce que** l'arbre (12) est mobile le long du dispositif de guidage (9) au moyen du dispositif de palier (15) de telle façon que le mélangeur (1, 1') peut passer d'une position de montage à une position d'intégration, dans lequel un angle entre l'axe longitudinal de l'arbre (12) et une verticale peut être agrandi pendant le passage du mélangeur (1, 1') de la position de montage à la position d'intégration.

2. Set selon la revendication 1, **caractérisé en ce que** l'angle entre l'axe longitudinal de l'arbre (12) et la verticale fait entre 5° et 60°, de préférence entre 10° et 50° lorsque le mélangeur (1, 1') est dans sa position d'intégration.

3. Set selon la revendication 1 ou 2, dans lequel l'au moins un élément de mélange (14, 14') est formé par un élément batteur (27) en forme de plaque qui est relié fixement à l'arbre (12) au moyen d'un dispositif de fixation (23), **caractérisé par** un dispositif d'articulation (24) au moyen duquel un angle qui est compris par l'arbre (12) et au moins un tronçon partiel du dispositif de fixation (23) peut être modifié.

4. Récipient, en particulier fermenteur (2) pour la fermentation de substances biodégradables, présentant au moins une plaque de fond (3), au moins une paroi (4) ainsi qu'au moins un dispositif de couvercle, dans lequel un intérieur (7) du récipient (2) est clos en étanchéité aux gaz au moyen de la plaque de fond (3), de la paroi (4) et du dispositif de couvercle, dans lequel le récipient (2) présente au moins un mélangeur (1, 1') au moyen duquel des substances se trouvant à l'intérieur (7) du récipient (2) peuvent être mélangées, dans lequel le dispositif de couvercle est formé par au moins deux unités de couvercle, à savoir au moins une unité de plaque (6, 6') et au moins une unité de membrane (5), dans lequel l'unité de plaque (6, 6') est formée par une pièce en forme de plaque et l'unité de membrane (5) est formée par une membrane, dans lequel l'unité de plaque (6, 6') et l'unité de membrane (5) sont reliées entre elles essentiellement en étanchéité aux gaz dans des parties dans lesquelles elles se touchent, dans lequel le récipient présente au moins un dispositif de guidage (9) au moyen duquel le dispositif de palier (15) du mélangeur (1, 1') peut être dirigé en direction du dispositif de réception (11) à la suite d'un passage du mélangeur (1, 1') d'une position de montage à une position d'intégration, dans lequel le dispositif de réception (11) est disposé à une extrémité du dispositif de guidage (9) et détermine la position d'intégration dans laquelle il bloque un mouvement involontaire du dispositif de palier (15), **caractérisé en ce que** le mélangeur (1, 1'), dans sa position d'intégration, est disposé incliné contre une verticale, dans lequel la verticale et un axe longitudinal d'un arbre (12) du mélangeur (1, 1') incluent un angle entre 5° et 60°.

5. Récipient (2) selon la revendication 4, **caractérisé en ce qu'**au moins une partie partielle de l'au moins une unité de plaque (6, 6') qui présente l'ouverture de montage (18), est dirigée parallèlement à l'axe longitudinal de l'arbre (12).

6. Récipient (2) selon la revendication 4 ou 5, **caractérisé en ce que** l'unité de plaque (6, 6') est alignée parallèlement à la plaque de fond (3).

7. Récipient (2) selon l'une des revendications 4 à 6, **caractérisé en ce que** l'unité de plaque (6, 6') présente au moins une ouverture de montage (18) à travers laquelle le mélangeur (1, 1') peut être introduit d'un environnement (8) de l'unité de plaque (6) dans l'intérieur (7) du fermenteur (2).

8. Récipient (2) selon la revendication 7, **caractérisé en ce que** l'ouverture de montage (18) est rectangulaire, dans lequel une section transversale libre de l'ouverture de montage (18) qui reste après une insertion du mélangeur (1, 1') dans le fermenteur (2) et relie l'intérieur (7) du fermenteur (2) à l'extérieur de l'unité de plaque (6, 6'), peut être fermée essentiellement en étanchéité aux gaz.

9. Récipient (2) selon la revendication 7 ou 8, **caractérisé en ce que** l'ouverture de montage (18) est disposée dans une distance mesurée horizontalement inférieure à 2 m, de préférence inférieure à 1,5 m, plus encore de préférence inférieure à 1 m, de la paroi (4) du fermenteur (2).

10. Récipient (2) selon l'une des revendications 4 à 9, **caractérisé en ce qu'**un tronçon d'extrémité supérieur du mélangeur (1, 1') détourné de la plaque de fond (3) du fermenteur (2) est relié à l'unité de plaque (6) en transmission de force.

11. Récipient (2) selon la revendication 4, présentant un dispositif de réception (11) relié à la plaque de fond (3), au moyen duquel un dispositif de palier (15) du mélangeur (1, 1'), qui est disposé dans une partie d'extrémité d'un arbre (12) du mélangeur (1, 1') tournée vers la plaque de fond (3) du récipient (2), peut être reçu, ainsi qu'un dispositif de guidage (9) au moyen duquel le dispositif de palier (15) du mélangeur (1, 1') peut être amené en direction du dispositif de réception (11) à la suite d'un passage du mélangeur (1, 1') d'une position de montage à une position d'intégration, dans lequel le dispositif de réception (11) est disposé à une extrémité du dispositif de guidage (9) et détermine la position d'intégration, dans laquelle il bloque un mouvement involontaire du dispositif de palier (15), **caractérisé en ce qu'**un angle entre la verticale et un axe longitudinal de l'arbre du mélangeur (1, 1') s'agrandit pendant le passage de la position de montage à la position d'intégration, de sorte que le mélangeur (1) est disposé incliné contre la position verticale dans sa position d'intégration.

12. Récipient selon la revendication 11, **caractérisé en ce que** la verticale et un axe longitudinal d'un arbre (12) du mélangeur (1) incluent un angle entre 5° et 60°, de préférence entre 10° et 50°.

13. Récipient selon la revendication 11 ou 12, **caractérisé en ce que** le dispositif de guidage (9) présente une unité de coulissement étirée en longueur ou une unité de déroulement étirée en longueur, de préférence un tube de guidage (10), dans lequel l'unité de coulissement est conçue de préférence lisse et l'unité de déroulement présente de préférence une face de déroulement concave ou convexe en section transversale.

14. Récipient (2) selon l'une des revendications 11 à 13, **caractérisé en ce que** le dispositif de guidage (9) présente dans un tronçon de guidage, un angle d'inclinaison par rapport à l'horizontale d'au moins 5°, de préférence d'au moins 7,5°, plus encore de préférence d'au moins 10°.

15. Récipient (2) selon l'une des revendications 11 à 14, **caractérisé en ce que** le dispositif de réception (11) est disposé dans une distance horizontale, mesurée verticalement par rapport à l'au moins une paroi d'au moins 1,5 m, de préférence d'au moins 2 m, plus encore de préférence d'au moins 2,5 m, de la paroi (4).

16. Procédé d'intégration d'un mélangeur (1, 1') dans un récipient, en particulier dans un fermenteur (2) comprenant les étapes de procédé suivantes :
a) un dispositif de palier (15) du mélangeur (1, 1'), qui est disposé sur une extrémité d'un arbre (12) du mélangeur (1, 1') tournée vers la plaque de fond (3) du récipient (2) et relié à celui-ci, est disposé dans un dispositif de réception (11) du récipient (2) relié à la plaque de fond (3) du récipient (2), dans lequel, pour insérer le dispositif de palier (15) du mélangeur (1, 1') dans le dispositif de réception (11), le dispositif de palier (15), partant d'une position de montage du mélangeur (1, 1'), est d'abord inséré dans un dispositif de guidage (9) et dirigé au moyen de ce dispositif de guidage (9) en direction du dispositif de réception (11) qui est disposé à une extrémité du dispositif de guidage (9), jusqu'à ce que le dispositif de palier (15) atteigne le dispositif de réception (11), soit reçu par celui-ci et que le mélangeur (1, 1') adopte ainsi sa position d'intégration.
b) le mélangeur (1, 1') est placé dans son tronçon de palier supérieur détourné de la plaque de fond (3),
**caractérisé par** l'étape de procédé suivante :
c) pendant le passage du mélangeur (1, 1') de la position de montage à la position d'intégration, un angle entre une verticale et un axe longitudinal de l'arbre (12) est agrandi.

17. Procédé selon la revendication 16, **caractérisé en ce que** le mélangeur (1, 1') est abaissé d'une distance maximale de 2 m mesurée verticalement à une face de paroi d'une paroi (4) du récipient (2), depuis une face supérieure du récipient (2) de préférence également verticalement, dans lequel, après que le dispositif de palier (15) a atteint sa position d'intégration, une extrémité du mélangeur (1, 1') détournée de la plaque de fond (3) du récipient (2) est déplacée verticalement à la face de paroi de la paroi (4) du récipient (2) en direction d'une partie centrale du récipient (2) puis fixée.

18. Procédé selon la revendication 16 ou 17, **caractérisé en ce que** le mélangeur (1') est abaissé dans le récipient (2) à travers une ouverture de montage (18) qui se trouve dans un dispositif de couvercle du récipient (2) et est ensuite fixé dans une ouverture de palier (31) qui se trouve dans une paroi (4) du récipient (2), de sorte que le mélangeur (1') adopte sa position de fonctionnement.
